# EUROPÄISCHE PATENTSCHRIFT

(11) **EP 3 315 986 B1**
(45) Veröffentlichungstag und Bekanntmachung des Hinweises auf die Patenterteilung: **17.02.2021**
(21) Anmeldenummer: 16196323.6
(22) Anmeldetag: 28.10.2016
(51) Int. Cl.: G01R 33/28, G16H 40/63, G01R 33/56, G01R 33/54, A61B 5/00, A61B 5/107

(54) **VERFAHREN ZUM BEREITSTELLEN EINER INFORMATION ZUR VORBEREITUNG EINER MR-BILDGEBUNG**
METHOD FOR PROVIDING INFORMATION FOR PREPARING AN MR IMAGING
PROCÉDÉ DE FOURNITURE D'UNE INFORMATION POUR PRÉPARER UNE IMAGÉRIE PAR RÉSONANCE MAGNÉTIQUE

(43) Veröffentlichungstag der Anmeldung: 02.05.2018
(73) Patentinhaber: Siemens Healthcare GmbH, 91052 Erlangen (DE)
(72) Erfinder: Rinck, Daniel, 91301 Forchheim (DE); Gaß, Verena, 90461 Nürnberg (DE)

(56) Entgegenhaltungen:
- EP-A2- 1 271 172
- WO-A1-2016/120073
- DE-A1-102006 052 711
- DE-A1-102014 207 020
- JP-A- 2004 201 977
- US-A1- 2013 279 779

## Beschreibung

Die Erfindung betrifft ein Verfahren zum Bereitstellen einer Vorbereitungsinformation zur Vorbereitung einer Magnetresonanz-Bildgebung eines Untersuchungsobjekts mittels eines Magnetresonanzgeräts, ein System und ein Computerprogrammprodukt.

In einem Magnetresonanzgerät, auch Magnetresonanztomographiesystem genannt, wird üblicherweise der zu untersuchende Körper einer Untersuchungsobjekts, beispielsweise eines Patienten, eines gesunden Probanden, eines Tiers oder eines Phantoms, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenschaltungen ausgespielt. Über eine Hochfrequenzantenneneinheit werden dann mittels geeigneter Antenneneinrichtungen Hochfrequenz-Pulse, beispielsweise Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Gerade die Vorbereitung der Magnetresonanz-Bildgebung, beispielsweise das Anlegen von zumindest einer Lokalspule und/oder die Lagerung des Untersuchungsobjekts auf der Patientenlagerungsvorrichtung des Magnetresonanzgeräts, kann längere Zeiten in Anspruch nehmen. Deshalb sind Automatisierungen und/oder Möglichkeiten zur Unterstützung der Vorbereitung der Magnetresonanz-Bildgebung wünschenswert. Beispielsweise können Verfahren zur automatischen Erkennung der Position der zumindest einen Lokalspule und/oder Optimierung der Positionierung des Untersuchungsobjekts von großem Interesse sein.

Dabei kann es beispielsweise wünschenswert sein, bereits vor dem Start der Magnetresonanz-Bildgebung eine Position der zumindest einen Lokalspule überprüfen zu können, um ggf. eine Optimierung der Position durchführen zu können. Dafür kann es wichtig sein, die Position der zumindest einen Lokalspule mit einer hohen Genauigkeit zu erkennen. Es sind Sensoren zur Detektion der Position der Lokalspule, wie beispielsweise B0-Hallsensoren, Ultraschallsensoren, RFID Tags, Kodierung der Spulen über eingelagerte Materialien mit spezifischem Magnetresonanz-Kontrast, bekannt. Diese bekannten Sensoren können üblicherweise allerdings nur Angaben zu einer punktuellen Position der zumindest einen Lokalspule machen. In den meisten Fällen kann mit diesen bekannten Sensoren, insbesondere wenn nur ein bekannter Sensor eingesetzt wird, nicht die Ausrichtung und/oder Verformung der zumindest einen Lokalspule im Raum zum Untersuchungsobjekt, gerade bei flexiblen Spulen, detektiert werden.

Auch kann es beispielsweise wünschenswert sein, bei der Vorbereitung der Magnetresonanz-Bildgebung automatisch eine Lokalisierung eines zu untersuchenden Organs im Untersuchungsobjekt zu bestimmen. Üblicherweise wird nämlich die zu untersuchende Körperregion, welche ungefähr das zu untersuchende Organ enthält, mit einem Laserkreuz an einer Vorderseite des Magnetresonanzgeräts positioniert und anschließend automatisch in ein Isozentrum des Magnetresonanzgeräts gefahren. Die Positionierung des zu untersuchenden Organs erfolgt also manuell, wobei das zu untersuchende Organ während der Positionierung typischerweise nicht sichtbar oder schwer zu ertasten ist.

Dieser Problemfall kann beispielsweise bei der Vorbereitung der Untersuchung der Prostata des Untersuchungsobjekts auftreten. Für die Positionierung der Prostata steht üblicherweise lediglich der Beckenkamm des Untersuchungsobjekts als tastbare Landmarke zu Verfügung. Daher bedarf es einer erfahrenen Bedienperson, um in diesem Fall manuelle Positionierung durchzuführen. Da die zumindest eine Lokalspule, welche zum Empfangen der Magnetresonanz-Signale aus der Prostata vorgesehen ist, nur ein begrenztes Empfangsprofil aufweist, kann es weiterhin notwendig sein, die zumindest eine Lokalspule möglichst nahe an der Prostata zu positionieren.

Aus der JP 2004 201977 A ist eine Verwendung einer stereoskopischen Kamera zum Erfassen einer dreidimensionalen Raumkoordinate als Referenz für eine folgende Magnetresonanz-Bildgebung bekannt.

Aus der DE 10 2014 207020 A1 ist ein Verfahren zur Positionierung einer Lokalspule zur Aufnahme von Magnetresonanz-Messdaten bekannt, wobei ein die Oberfläche des bereits zur Aufnahme auf einer Patientenliege der Magnetresonanzeinrichtung positionierten, aufzunehmenden Patienten beschreibender Oberflächendatensatz gemessen wird, der Oberflächenverlauf des Patienten aus dem Oberflächendatensatz extrahiert wird und wenigstens eine Position und/oder Form wenigstens einer der wenigstens einen Lokalspule in Abhängigkeit von dem Oberflächenverlauf so gewählt wird, dass ein vorbestimmter Abstand zwischen der Oberfläche des Patienten und der Oberfläche der Lokalspule besteht.

Aus der DE 10 2006 052711 A1 ist ein Verfahren zur Oberflächenerfassung einer Untersuchungsperson, die auf einer Patientenliege einer medizinischen Diagnoseeinrichtung angeordnet ist, bekannt, wobei eine auf den Patienten angestrahlte Lichtlinie mittels einer Kamera erfasst wird, um beispielsweise zur Erfassen, ob Lokalspulen an den richtigen Patientenstellen positioniert sind.

WO 2016/120073 A1 ist ein Erfassen eines Tiefenbildes mit einer Kamera eines Magnetresonanzgeräts vor dem Positionieren des Untersuchungsobjekts im Aufnahmebereich zur Vorbereitung der Magnetresonanz-Bildgebung bekannt.

Der Erfindung liegt die Aufgabe zu Grunde, eine besonders vorteilhafte Vorbereitung einer Magnetresonanz-Bildgebung zu ermöglichen.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren zum Bereitstellen einer Vorbereitungsinformation zur Vorbereitung einer Magnetresonanz-Bildgebung eines Untersuchungsobjekts mittels eines Magnetresonanzgeräts, umfassend folgende Verfahrensschritte:
- Lagerung des Untersuchungsobjekts auf einer Patientenlagerungsvorrichtung des Magnetresonanzgeräts,
- Erfassen einer Tiefenkarte des auf der Patientenlagerungsvorrichtung gelagerten Untersuchungsobjekts mittels einer Tiefenkamera,
- Ermitteln einer Vorbereitungsinformation zur Vorbereitung der Magnetresonanz-Bildgebung unter Verwendung der erfassten Tiefenkarte und
- Bereitstellen der Vorbereitungsinformation, wobei das Ermitteln der Vorbereitungsinformation ein Berechnen einer Lokalisierungswahrscheinlichkeit eines in der Magnetresonanz-Bildgebung zu untersuchenden Organs in einem Körper des Untersuchungsobjekts unter Verwendung der Tiefenkarte umfasst, wobei das Berechnen der Lokalisierungswahrscheinlichkeit des zu untersuchenden Organs eine Anpassung eines Körpermodells, welches eine Information über eine Lokalisierung des zu untersuchenden Organs im Körpermodell umfasst, an das Untersuchungsobjekt unter Verwendung der Tiefenkarte umfasst.

Das Erfassen der Tiefenkarte und das Ermitteln und Bereitstellen der Vorbereitungsinformation erfolgen insbesondere zeitlich nach der Lagerung des Untersuchungsobjekts auf der Patientenlagerungsvorrichtung des Magnetresonanzgeräts. Das Untersuchungsobjekt wird insbesondere so auf der Patientenlagerungsvorrichtung gelagert, so dass die Magnetresonanz-Bildgebung vom Untersuchungsobjekt durchgeführt werden kann, nachdem das Untersuchungsobjekt mittels eines Verfahrens der Patientenlagerungsvorrichtung in einem Isozentrum des Magnetresonanzgeräts positioniert worden ist. Selbstverständlich kann zwischen einzelnen Verfahrensschritten auch eine Umlagerung des Untersuchungsobjekts auf der Patientenlagerungsvorrichtung erfolgen. Nach der Umlagerung des Untersuchungsobjekts kann ein erneutes Erfassen der Tiefenkarte und/oder ein Erneutes Ermitteln und Bereitstellen der Vorbereitungsinformation sinnvoll sein.

Die Tiefenkarte wird vom Untersuchungsobjekt vorteilhafterweise zeitlich vor der Positionierung des Untersuchungsobjekts im Isozentrum des Magnetresonanzgeräts erfasst. Die Tiefenkarte wird also insbesondere erfasst, während das Untersuchungsobjekt noch zumindest teilweise außerhalb einer tunnelförmigen Öffnung des Magnetresonanzgeräts positioniert ist. Gleichermaßen kann auch die Vorbereitungsinformation zeitlich vor der Positionierung des Untersuchungsobjekts im Isozentrum des Magnetresonanzgeräts ermittelt und bereitgestellt werden. Selbstverständlich ist es in bestimmten Anwendungsfällen auch denkbar, dass die Tiefenkarte zeitlich nach dem Positionieren des Untersuchungsobjekts im Isozentrum des Magnetresonanzgeräts erfasst wird, beispielsweise um eine weitere Überwachung des Untersuchungsobjekts während der Magnetresonanz-Bildgebung, insbesondere wenn das Untersuchungsobjekt mit den Füßen voran in das Magnetresonanzgerät verfahren wurde (Feet-First Untersuchung), zu ermöglichen. Auch die Vorbereitungsinformation kann zeitlich nach dem Positionieren des Untersuchungsobjekts im Isozentrum des Magnetresonanzgeräts erfasst ermittelt und/oder bereitgestellt werden.

Die Tiefenkamera kann insbesondere eine dreidimensionale Tiefenkarte des Untersuchungsobjekts erfassen. Die Tiefenkarte kann derart eine zweidimensionale Matrix umfassen, welche den Abstand von der abgebildeten Szene zu einem Punkt oder eine Ebene, welche typischerweise mit der Tiefenkamera assoziiert sind, kodiert. Daher umfasst die Tiefenkarte insbesondere Bildpunkte, welche einen Abstand des abgebildeten Objekts zu der Tiefenkamera kodieren. Insbesondere bei einer geeigneten Kalibrierung der Tiefenkamera, können die Bildpunkte der Tiefenkarte in physikalischen Abstandseinheiten, wie beispielsweise Meter, abgespeichert bzw. angezeigt werden. Wenn die Tiefenkarte, exemplarisch als Grauwertbild, angezeigt wird, kann insbesondere ein näher an der Tiefenkamera positioniertes Objekt, eventuell eine auf dem Untersuchungsobjekt positionierte Lokalspule, in der Tiefenkarte dunkler erscheinen, als ein weiter entferntes Bildelement, beispielsweise ein Randbereich des Untersuchungsobjekts und/oder eine Oberfläche der Patientenlagerungsvorrichtung und/oder ein Boden des Untersuchungsraums. Die Tiefenkarte kann derart detailliert eine Oberfläche des Untersuchungsobjekts und ggf. von auf dem Untersuchungsobjekt positionierten weiteren Objekten, wie beispielsweise einer Lokalspule oder einer Decke, abbilden.

Die Tiefenkamera, die zum Erfassen der Tiefenkarte eingesetzt wird, kann einen geeigneten Tiefensensor umfassen. Nur exemplarisch sind einige mögliche Tiefensensoren für die Tiefenkamera genannt: ein Time-of-Flight Sensor (TOF-Sensor), ein Stereo-Triangulationssensor bzw. ein Setup mit mehreren Kameras, ein Sensor zum Erkennen eines strukturierten Lichtmusters, welches auf das Untersuchungsobjekt projiziert wird (beispielsweise von Infrarotlicht, wie bei der Microsoft Kinect Kamera). Eine genaue Ausbildung dieser Tiefensensoren bzw. mögliche weitere Tiefensensoren sind dabei dem Fachmann bekannt, so dass hier nicht genauer auf diese eingegangen werden soll.

Die Tiefenkamera ist insbesondere in dem Untersuchungsraum, in welchem sich auch das Magnetresonanzgerät befindet, positioniert. Für das Erfassen der Tiefenkarte ist die Tiefenkamera vorteilhafterweise direkt über der Patientenlagerungsvorrichtung, sofern diese außerhalb der tunnelförmigen Öffnung des Magnetresonanzgeräts eingestellt ist, positioniert. Dabei kann die Tiefenkamera an der Decke des Untersuchungsraums montiert sein. Die Tiefenkamera kann derart senkrecht von oben die Tiefenkarte des Untersuchungsobjekts akquirieren. Selbstverständlich kann auch eine schräge bzw. versetzte Positionierung der Tiefenkamera sinnvoll sein. Mögliche weitere Montierungen der Tiefenkamera im Untersuchungsraum, beispielsweise an einer Wand und/oder auf einem Ständer und/oder bewegliche Montierungen, sind selbstverständlich denkbar.

Das Erfassen der Tiefenkarte des Untersuchungsobjekts kann bedeuten, dass die Tiefenkarte von dem Untersuchungsobjekt alleine oder von dem Untersuchungsobjekt zusammen mit zumindest einer weiteren Komponente, welche in räumlicher Nähe zu dem Untersuchungsobjekt positioniert ist, erfasst wird. Die zumindest eine weitere Komponente kann insbesondere zumindest eine Lokalspule, welche zum Empfangen von Magnetresonanz-Signalen während der Magnetresonanz-Bildgebung des Untersuchungsobjekts vorgesehen ist, umfassen. Derart kann die Tiefenkarte das Untersuchungsobjekt zusammen mit der zumindest einen Lokalspule abbilden. Die zumindest eine weitere Komponente kann auch eine Lagerungshilfe und/oder eine Decke umfassen. Es ist auch denkbar, dass die Tiefenkarte lediglich die zumindest eine Lokalspule abbildet. Generell erfasst die Tiefenkamera also insbesondere die Tiefenkarte von einer Untersuchungsszene, welche das Untersuchungsobjekt und/oder die Patientenlagerungsvorrichtung und/oder zumindest eine Komponente, wie beispielsweise zumindest eine Lokalspule, umfassen kann. Die Tiefenkamera kann zusätzlich auch ein RGB-Bild und/oder ein Infrarot-Bild von der Untersuchungsszene erfassen. Das RGB-Bild und/oder Infrarot-Bild kann dann ebenfalls zum Ermitteln der Vorbereitungsinformation, beispielsweise über eine Kantendetektion der zumindest einen Lokalspule im RGB-Bild und/oder Infrarot-Bild, verwendet werden.

Die Vorbereitungsinformation wird insbesondere mittels eines Ermittlungsalgorithmus ermittelt, welcher als Eingangsparameter die erfasste Tiefenkarte und/oder von der erfassten Tiefenkarte abgeleitete Informationen und als Ausgangsparameter die Vorbereitungsinformation umfasst. Selbstverständlich können weitere, dem Fachmann als sinnvoll erscheinende Eingangsparameter in das Ermitteln der Vorbereitungsinformation eingehen. Die Vorbereitungsinformation ist insbesondere auf die Vorbereitung der Magnetresonanz-Bildgebung des Untersuchungsobjekts bezogen. Beispielsweise kann das Bereitstellen der Vorbereitungsinformation die Vorbereitung der Magnetresonanz-Bildgebung unterstützen und/oder vereinfachen und/oder automatisieren. Unter der Vorbereitung der Magnetresonanz-Bildgebung sind insbesondere Handlungen zu verstehen, welche zeitlich vor dem Start der Akquisition der Magnetresonanz-Signale, insbesondere zeitlich vor dem Positionieren des Untersuchungsobjekts im Isozentrum des Magnetresonanzgeräts, erfolgen. Beispielsweise kann die Vorbereitung des Untersuchungsobjekts die korrekte Lagerung des Untersuchungsobjekts auf der Patientenlagerungsvorrichtung und/oder eine geeignete Positionierung von zumindest einer Lokalspule auf dem Untersuchungsobjekt umfassen. Die Vorbereitungsinformation kann konkret auf diese Handlungen bezogen sein.

Das Bereitstellen der Vorbereitungsinformation kann eine Ausgabe der Vorbereitungsinformation, beispielsweise auf einer Anzeigeeinheit und/oder mittels einer Projektionseinheit und/oder mittels einer Audioeinheit umfassen. Alternativ oder zusätzlich kann das Bereitstellen der Vorbereitungsinformation ein Abspeichern der Vorbereitungsinformation in einer Datenbank umfassen. Alternativ oder zusätzlich kann das Bereitstellen der Vorbereitungsinformation eine Weitergabe der Vorbereitungsinformation an eine Steuereinheit umfassen, welche basierend auf der Vorbereitungsinformation automatisch und/oder semiautomatisch die Vorbereitung der Magnetresonanz-Bildgebung steuern kann.

Die Vorbereitungsinformation kann derart wertvolle Zusatzinformationen zur Vorbereitung der Magnetresonanz-Bildgebung bereitstellen bzw. zur zumindest teilweisen Automatisierung der Vorbereitung der Magnetresonanz-Bildgebung beitragen. Dabei kann die erfasste Tiefenkarte wertvolle Tiefeninformationen als Grundlage des Erstellens der Vorbereitungsinformation bereitstellen. So kann beispielsweise das beschriebene Vorgehen die Vorbereitung der Magnetresonanz-Bildgebung des Untersuchungsobjekts gerade für weniger geschultes bzw. unerfahrenes Personal erleichtern oder ermöglichen. Auch kann die Vorbereitungsinformation eine standardisierte Durchführung der Vorbereitung der Magnetresonanz-Bildgebung mit gleich bleibender Qualität, insbesondere für weniger erfahrenes Bedienpersonal, ermöglichen.

Erfindungsgemäß umfasst das Ermitteln der Vorbereitungsinformation ein Berechnen einer Lokalisierungswahrscheinlichkeit eines in der Magnetresonanz-Bildgebung zu untersuchenden Organs in einem Körper des Untersuchungsobjekts unter Verwendung der Tiefenkarte umfasst.

Das zu untersuchende Organ soll insbesondere für die Magnetresonanz-Bildgebung im Isozentrum des Magnetresonanzgeräts positioniert werden. Das zu untersuchende Organ wird insbesondere durch eine diagnostische Fragestellung festgelegt, welche mittels der in der Magnetresonanz-Bildgebung akquirierten Magnetresonanz-Bilddaten geklärt werden soll. Soll beispielsweise anhand der Magnetresonanz-Bilddaten ein Staging bzw. eine Erkennung eines Prostatakarzinoms durchgeführt werden, so ist in diesem Fall das zu untersuchende Organ die Prostata des Untersuchungsobjekts. Die in der Magnetresonanz-Bildgebung akquirierten Magnetresonanz-Bilddaten sollen demnach primär das zu untersuchende Organ und eventuell umliegende Organstrukturen abbilden. Das zu untersuchende Organ kann auch einen Körperbereich des Untersuchungsobjekts, beispielsweise einen Kopfbereich, einen Thoraxbereich oder einen Beckenbereich des Untersuchungsobjekts umfassen.

Die Lokalisierungswahrscheinlichkeit kann eine Wahrscheinlichkeitsverteilung einer räumlichen Lokalisierung des zu untersuchenden Organs im Körper des Untersuchungsobjekts umfassen. Die Lokalisierungswahrscheinlichkeit kann auch eine Information über eine Position einer höchsten Aufenthaltswahrscheinlichkeit des zu untersuchenden Organs im Körper des Untersuchungsobjekts umfassen. Die Lokalisierungswahrscheinlichkeit kann demnach eine Information bereitstellen, an weleher Position sich im konkreten Fall das zu untersuchende Organ im Körper des Untersuchungsobjekts am wahrscheinlichsten befindet. Derart kann die Lokalisierungswahrscheinlichkeit auch als Aufenthaltswahrscheinlichkeit des zu untersuchenden Organs bezeichnet werden. Dabei wird die Lokalisierungswahrscheinlichkeit vorzugsweise in Bezug auf ein fixes Koordinatensystem, beispielsweise ein Koordinatensystem des Magnetresonanzgeräts und/oder der Patientenlagerungsvorrichtung und/oder der Tiefenkamera berechnet. Die Lokalisierungswahrscheinlichkeit kann auch in Bezug auf ein Koordinatensystem, welches dem Untersuchungsobjekt, beispielsweise über Landmarken, zugeordnet ist, berechnet werden.

Für die Berechnung der Lokalisierungswahrscheinlichkeit kann die erfasste Tiefenkarte besonders vorteilhaft eingesetzt werden. So ist es denkbar, dass in der Tiefenkarte Konturen einer Oberfläche des Untersuchungsobjekts erkannt werden, anhand welcher die Lokalisierungswahrscheinlichkeit des zu untersuchenden Organs berechnet wird. Dafür kann, wie in einem der folgenden Abschnitte noch genauer beschreiben, ein Körpermodell an das Untersuchungsobjekt unter Verwendung der Tiefenkarte angepasst werden. Die berechnete Lokalisierungswahrscheinlichkeit kann als Vorbereitungsinformation bereitgestellt werden. Verschiedene Möglichkeiten dazu sind in einem der folgenden Abschnitte beschrieben.

Erfindungsgemäß umfasst das Berechnen der Lokalisierungswahrscheinlichkeit des zu untersuchenden Organs eine Anpassung eines Körpermodells, welches eine Information über eine Lokalisierung des zu untersuchenden Organs im Körpermodell umfasst, an das Untersuchungsobjekt unter Verwendung der Tiefenkarte.

Das Körpermodell, auch Avatar-Modell genannt, ist insbesondere als dreidimensionales Körpermodell ausgebildet. Das Körpermodell kann ein Standard-Modell eines menschlichen Körpers umfassen, welches beispielsweise basierend auf einer Mittelung über mehrere von dem Untersuchungsobjekt verschiedene Subjekte gebildet wird. Auch kann zur Generierung des Körpermodells auf größere Datenbanken, welche Modellierungen des menschlichen Körpers in Schnittbildaufnahmen umfassen, zurückgegriffen werden. Derart kann das Körpermodell einen "Normmenschen" repräsentieren. Das Körpermodell kann auch basierend auf patientenspezifischen Parametern, wie beispielsweise Geschlecht und/oder Größe und/oder Alter und/oder Gewicht des Untersuchungsobjekts, geeignet aus mehreren möglichen Körpermodellen ausgewählt werden oder ausgehend von dem Standard-Modell angepasst werden. Derart kann eine Registrierung des Untersuchungsobjekts für die Magnetresonanz-Bildgebung zumindest teilweise automatisiert werden.

Das Körpermodell kann Konturinformationen des menschlichen Körpers umfassen, so dass die Anpassung des Körpermodells an das Untersuchungsobjekt eine Angleichung der Konturinformationen des Körpermodells an eine in der Tiefenkarte erkannte Oberfläche des Untersuchungsobjekts umfasst. Alternativ oder zusätzlich können auch in der Tiefenkarte identifizierte Landmarken zur Anpassung des Körpermodells, in welchem diese Landmarken ebenfalls definiert sind, an das Untersuchungsobjekt verwendet werden. Beispielsweise können als Landmarken ein Schultergelenk, eine Hüfte, ein Knie, eine Hand, ein Ellenbogen, usw. im Körpermodell definiert sein. Die Anpassung des Körpermodells an das Untersuchungsobjekt kann demnach basierend auf einer Anpassung der Landmarken des Körpermodells an die in der Tiefenkarte identifizierten Landmarken erfolgen. Hierfür kann auch das Körpermodell in mehrere Körpersegmente aufgeteilt sein, wobei eine individuelle Anpassung der mehreren Körpersegmente an das Untersuchungsobjekt erfolgen kann. Weitere Anpassungsverfahren (Matching-Verfahren) sind selbstverständlich denkbar. Anhand der Anpassung des Körpermodells an das Untersuchungsobjekts kann eine Identifizierung einer Körperoberfläche des Untersuchungsobjekts in der Tiefenkarte durchgeführt werden. Durch die Anpassung des Körpermodells an das Untersuchungsobjekt kann die individuelle Anatomie des Untersuchungsobjekts berücksichtigt werden. Für die Anpassung des Körpermodells an die Tiefenkarte wird die Tiefenkarte vorteilhafterweise vor einer Positionierung von einer Komponente auf dem Untersuchungsobjekt, wie beispielsweise einer Lokalspule, aufgenommen.

Die Information über die Lokalisierung des zu untersuchenden Organs im Körpermodell, welche vom Körpermodell umfasst wird, ist insbesondere in Bezug zu den Konturinformationen und/oder Landmarken des Körpermodells hinterlegt. Derart kann basierend auf der Anpassung des Körpermodells an das Untersuchungsobjekt die Information über die Lokalisierung des zu untersuchenden Organs im Körpermodell an die patientenspezifischen Gegebenheiten des Untersuchungsobjekts angepasst werden. Derart kann die Lokalisierungswahrscheinlichkeit des zu untersuchenden Organs besonders einfach basierend auf der Anpassung des Körpermodells berechnet werden. Die Information über die Lokalisierung des zu untersuchenden Organs im Körpermodell, welche bei der Anpassung des Körpermodells spezifisch an das Untersuchungsobjekt angepasst wird, kann nämlich eine besonders vorteilhafte Grundlage zur Berechnung der Lokalisierungswahrscheinlichkeit des zu untersuchenden Organs im Körper des Untersuchungsobjekts darstellen.

Weiterhin kann das an das Untersuchungsobjekt angepasste Körpermodell eine Grundlage zur automatischen oder semiautomatischen Bestimmung von zumindest einem patientenspezifischen und/oder untersuchungsspezifischen Parameter sein. Der zumindest eine solche Parameter kann beispielsweise eine Orientierung einer Lagerung des Untersuchungsobjekts auf der Patientenlagerungsvorrichtung und/oder eine Größe des Untersuchungsobjekts und/oder ein Gewicht des Untersuchungsobjekts sein. Der zumindest eine Parameter kann nach der Bestimmung automatisch an eine Steuereinheit des Magnetresonanzgeräts übermittelt werden, so dass eine manuelle Eingabe des zumindest einen Parameters vorteilhafterweise entfallen kann. Derart kann das Anpassen des Körpermodells an das Untersuchungsobjekt eine vorteilhafte Doppelfunktion ausfüllen, nämlich gleichzeitig zum Berechnen der Lokalisierungswahrscheinlichkeit des zu untersuchenden Organs und zum Bestimmen des zumindest einen patientenspezifischen und/oder untersuchungsspezifischen Parameters dienen.

Eine Ausführungsform sieht vor, dass das Bereitstellen der Vorbereitungsinformation eine Anzeige von Anzeigedaten, welche basierend auf der Lokalisierungswahrscheinlichkeit des zu untersuchenden Organs erstellt werden, auf einer Anzeigeeinheit zusammen mit einer Darstellung einer Repräsentation eines menschlichen Körpers umfasst.

Die Anzeigeeinheit ist vorteilhafterweise im Untersuchungsraum, in welchem sich auch das Magnetresonanzgerät und die Tiefenkamera befindet, lokalisiert. Die Anzeigeeinheit kann einen dem Magnetresonanzgerät, insbesondere der Patientenlagerungsvorrichtung, zugeordneten Bildschirm umfassen. Alternativ oder zusätzlich kann die Anzeigeeinheit ein Touch-Interface umfassen, welcher kabellos oder kabelgebunden mit dem Magnetresonanzgerät verbunden ist.

Die Repräsentation des menschlichen Körpers kann besonders vorteilhaft spezifisch an das Untersuchungsobjekt, beispielsweise anhand von patientenspezifischen Parametern und/oder der akquirierten Tiefenkarte, erstellt werden. Die Anzeigedaten können eine Information umfassen, an welcher Stelle bezüglich der Repräsentation des menschlichen Körpers die höchste Lokalisierungswahrscheinlichkeit des zu untersuchenden Organs vorliegt. Es ist alternativ oder zusätzlich auch denkbar, dass als Anzeigedaten ein Konfidenzradius für die Lokalisierungswahrscheinlichkeit des zu untersuchenden Organs auf der Repräsentation des menschlichen Körpers dargestellt wird, wie es in einem der folgenden Abschnitte analog für die Projektionsdaten genauer beschrieben wird.

Eine Ausführungsform sieht vor, dass das Bereitstellen der Lokalisierungswahrscheinlichkeit eine Projektion von Projektionsdaten, welche basierend auf der Lokalisierungswahrscheinlichkeit des zu untersuchenden Organs erstellt werden, auf eine Körperoberfläche des Untersuchungsobjekts mittels einer Projektionseinheit umfasst.

Die Projektionseinheit ist vorteilhafterweise im Untersuchungsraum, in welchem sich auch das Magnetresonanzgerät und die Tiefenkamera befindet, lokalisiert. Die Projektionseinheit kann in einer räumlichen Nähe zu der Tiefenkamera installiert sein und/oder in einer kombiniert in einer Vorrichtung mit der Tiefenkamera ausgebildet sein. Vorteilhafterweise ist die Projektionseinheit an einer Decke des Untersuchungsraums, insbesondere senkrecht über der Patientenlagerungsvorrichtung, lokalisiert. Die Projektionseinheit ist insbesondere zur Projektion der Projektionsdaten auf das Untersuchungsobjekt und/oder auf die Patientenlagerungsvorrichtung ausgebildet. Dafür kann die Projektionseinheit dem Fachmann als sinnvoll erscheinende Projektionsmittel, beispielsweise Laser-Projektionsmittel, umfassen.

Die Projektionsdaten können eine Information umfassen, an welcher Stelle bezüglich des Körpers des Untersuchungsobjekts die höchste Lokalisierungswahrscheinlichkeit des zu untersuchenden Organs vorliegt. Derart kann die höchste Lokalisierungswahrscheinlichkeit des zu untersuchenden Organs direkt auf die entsprechende Stelle auf die Oberfläche des Untersuchungsobjekts projiziert werden. Insbesondere können die Projektionsdaten dabei auf die zu dem zu untersuchenden Organ korrespondierende Körperoberfläche des Untersuchungsobjekts projiziert werden. Es ist auch denkbar, dass die Projektionsdaten eine geeignete Position für eine Lokalspule Empfangen von Magnetresonanz-Signalen aus dem zu untersuchenden Organ wiedergeben, wobei die geeignete Position für die Lokalspule anhand der Lokalisierungswahrscheinlichkeit des zu untersuchenden Organs ermittelt wird.

Die Anzeigedaten und/oder Projektionsdaten können beispielsweise wertvolle Informationen bereitstellen, an welche Stelle eine Lokalspule für das Empfangen von Magnetresonanz-Signalen aus dem zu untersuchenden Organ positioniert werden muss. Auch können die Anzeigedaten und/oder Projektionsdaten wertvolle Informationen zur Positionierung des zu untersuchenden Organs im Isozentrum des Magnetresonanzgeräts bereitstellen.

Eine Ausführungsform sieht vor, dass als Projektionsdaten zumindest ein Konfidenzradius auf die Körperoberfläche des Untersuchungsobjekts projiziert wird, wobei der zumindest eine Konfidenzradius die Lokalisierungswahrscheinlich des zu untersuchenden Organs wiederspiegelt.

Der Konfidenzradius kann einen Bereich auf der Körperoberfläche des Untersuchungsobjekts identifizieren, bei dem eine hohe Lokalisierungswahrscheinlichkeit des zu untersuchenden Organs vorliegt. Zur Bestimmung des Konfidenzradius kann die räumliche Verteilung der Lokalisierungswahrscheinlichkeit des zu untersuchenden Organs beispielsweise mit einem Schwellwert für die Lokalisierungswahrscheinlichkeit verglichen werden. Es ist auch denkbar, dass mehrere Konfidenzradien auf die Körperoberfläche des Untersuchungsobjekts projiziert werden, welche zu unterschiedlichen Höhen der Lokalisierungswahrscheinlichkeit des zu untersuchenden Organs korrespondieren. So können ein erster Konfidenzradius und ein zweiter Konfidenzradius projiziert werden, wobei der erste Konfidenzradius einen Bereich mit der höchsten Lokalisierungswahrscheinlichkeit des zu untersuchenden Organs begrenzt und der zweite Konfidenzradius einen Bereich mit einer geringeren Lokalisierungswahrscheinlichkeit des zu untersuchenden Organs begrenzt. Die Projektion des zumindest einen Konfidenzradius kann eine besonders aussagekräftige Darstellung der Lokalisierung des zu untersuchenden Organs ermöglichen.

Eine Ausführungsform sieht vor, dass anhand der ermittelten Lokalisierungswahrscheinlichkeit die Patientenlagerungsvorrichtung des Magnetresonanzgeräts derart verfahren wird, dass für die Magnetresonanz-Bildgebung das zu untersuchende Organ automatisch in einem Isozentrum des Magnetresonanzgeräts positioniert wird.

Die Patientenlagerungsvorrichtung wird insbesondere dann erst verfahren, wenn die weitere Vorbereitung der Magnetresonanz-Bildgebung abgeschlossen ist, also beispielsweise eine Lokalspule zum Erfassen der Magnetresonanz-Signale an einer geeigneten Position positioniert worden ist. Das automatische Positionieren des zu untersuchenden Organs im Isozentrum des Magnetresonanzgeräts kann automatisch oder manuell durch eine Bedienperson, beispielsweise mittels einer Betätigung eines Schalters, einer Eingabe in einem Touch-Interface oder mittels einer Gestensteuerung, ausgelöst werden. Die Patientenlagerungsvorrichtung kann dann insbesondere derart verfahren werden, dass der Bereich mit der höchsten ermittelten Lokalisierungswahrscheinlichkeit des zu untersuchenden Organs im Isozentrum des Magnetresonanzgeräts positioniert wird.

Das Positionieren des zu untersuchenden Organs im Isozentrum des Magnetresonanzgeräts ist wichtig, damit in der Magnetresonanz-Bildgebung Magnetresonanz-Bilddaten mit hoher Qualität von dem zu untersuchenden Organ akquiriert werden können. Die automatische Positionierung des zu untersuchenden Organs im Isozentrum des Magnetresonanzgeräts kann eine Erleichterung bzw. Beschleunigung der Vorbereitung der Magnetresonanz-Bildgebung darstellen. Die bekannte manuelle Positionierung des zu untersuchenden Organs mittels eines Laserkreuzes kann demnach nämlich entfallen. Diese kann nämlich zu Fehlpositionierungen führen, da die Anatomie des Untersuchungsobjekts häufig durch weitere Komponenten, wie beispielsweise einer Lokalspule oder einer Decke, verdeckt ist. So kann häufig bei der manuellen Positionierung mittels des Laserkreuzes lediglich eine auf dem Untersuchungsobjekt positionierte Lokalspule als Referenzobjekt verwendet werden. Liegt das Zentrum der Lokalspule allerdings nicht genau über dem zu untersuchenden Organ, so kann es vorkommen, dass bei der manuellen Positionierung das zu untersuchende Organ nicht direkt im Isozentrum des Magnetresonanzgeräts positioniert wird. Mittels des vorgeschlagenen automatischen Vorgehens kann demnach eine korrekte Positionierung des zu untersuchende Organs im Isozentrum des Magnetresonanzgeräts, insbesondere auch für ein Bedienpersonal mit weniger Erfahrung, sichergestellt werden.

Eine Ausführungsform sieht vor, dass die erfasste Tiefenkarte eine Lokalspule, welche zum Empfangen von Magnetresonanz-Signalen während der Magnetresonanz-Bildgebung des Untersuchungsobjekts vorgesehen ist, abbildet, wobei das Ermitteln der Vorbereitungsinformation ein Ermitteln einer Position der Lokalspule anhand der Tiefenkarte umfasst.

Die zum Empfangen der Magnetresonanz-Signale vorgesehene Lokalspule wird insbesondere für die Magnetresonanz-Untersuchung von einem Bedienpersonal auf einen zu untersuchenden Körperbereich des Untersuchungsobjekts angelegt. Die Lokalspule kann zum Empfangen von Magnetresonanz-Signalen mit einem höheren Signal-zu-Rauschverhältnis (SNR) als eine fest im Magnetresonanzgerät installierte Ganzkörperspule (Body Coil) ausgebildet sein, insbesondere da sie näher an dem zu untersuchenden Körperbereich des Untersuchungsobjekts positioniert ist. Es sind verschiedene Ausprägungen der Lokalspule denkbar, beispielsweise als Körperantenneneinheit, als Kopfantenneneinheit, als Schulterantenneneinheit, als Knieantenneneinheit, usw.

Für den vorliegenden Anwendungsfall ist die Lokalspule insbesondere an mehreren Stellen in Bezug auf das Untersuchungsobjekt bzw. die Patientenlagerungsvorrichtung positionierbar. Beispielsweise kann es sich bei der Lokalspule als flexible Körperantenneneinheit handeln, welche an unterschiedlichen Positionen auf dem Untersuchungsobjekt positioniert werden kann und auch unterschiedliche Formen annehmen kann. Dann kann das Ermitteln der Position der flexiblen Körperantenneneinheit zusätzlich auch ein Ermitteln einer Form der flexiblen Körperantenneneinheit umfassen.

Die Tiefenkarte wird insbesondere dann erfasst, wenn die Lokalspule bereits vom Bedienpersonal auf den zu untersuchenden Körperbereich des Untersuchungsobjekts angelegt wurde. Die erfasste Tiefenkarte kann eine vorteilhafte Grundlage zum Ermitteln der Position der Lokalspule darstellen. Ein Grund dafür ist, dass die Lokalspule sich typischerweise in der Tiefenkarte von der Körperoberfläche des Untersuchungsobjekts abhebt. Derart kann die Position der Lokalspule besonders einfach in der Tiefenkarte erkannt werden. Das Bereitstellen der Vorbereitungsinformation kann dann ein Bereitstellen der Position der Lokalspule umfassen. Vorteilhafterweise kann die erkannte Position der Lokalspule geeignet für eine Unterstützung der Vorbereitung der Magnetresonanz-Bildgebung verwendet werden.

In bestimmten Anwendungsfällen ist es auch denkbar, dass die erkannte Position der Lokalspule zur automatischen Detektion von potentiellen Kollisionen von Teilen der Lokalspule mit anderen Komponenten des Magnetresonanzgeräts, beispielsweise einer Begrenzung der tunnelförmigen Öffnung des Magnetresonanzgeräts, eingesetzt wird. Wenn eine solche potentielle Kollision detektiert wird, kann beispielsweise eine Warnung an das Bedienpersonal ausgegeben werden und/oder ein Verfahren der Patientenlagerungsvorrichtung angehalten bzw. verlangsamt werden. Es ist in bestimmten Anwendungsfällen auch denkbar, dass die Position der Lokalspule mittels Deep-Learning Verfahren anhand der Tiefenkarte erkannt wird.

Eine Ausführungsform sieht vor, dass das Erfassen der Tiefenkarte ein Erfassen einer ersten Tiefenkarte zeitlich vor einer Positionierung der Lokalspule für die Magnetresonanz-Bildgebung und ein Erfassen einer zweiten Tiefenkarte zeitlich nach der Positionierung der Lokalspule für die Magnetresonanz-Bildgebung umfasst, wobei das Ermitteln der Position der Lokalspule eine Bestimmung von Abweichungen zwischen der ersten Tiefenkarte und der zweiten Tiefenkarte umfasst.

Die Positionierung der Lokalspule für die Magnetresonanz-Bildgebung umfasst insbesondere das Anlegen der zum Empfangen der Magnetresonanz-Signale vorgesehenen Lokalspule auf einen zu untersuchenden Körperbereich des Untersuchungsobjekts von einem Bedienpersonal. Die erste Tiefenkarte wird insbesondere weiterhin nach der Lagerung des Untersuchungsobjekts auf der Patientenlagerungsvorrichtung erfasst. Derart kann die zweite Tiefenkarte Tiefeninformationen von der auf dem Untersuchungsobjekt positionierten Lokalspule aufweisen, während die Tiefeninformationen von der Lokalspule in der ersten Tiefenkarte noch nicht vorliegen.

Demnach können mittels der Bestimmung der Abweichungen zwischen der ersten Tiefenkarte und der zweiten Tiefenkarte Informationen erhalten werden, aus welchen sich die Position der Lokalspule ermitteln lässt. Vorteilhafterweise kann die Bestimmung der Abweichungen zwischen der ersten Tiefenkarte und der zweiten Tiefenkarte eine Differenzbildung zwischen der ersten Tiefenkarte und der zweiten Tiefenkarte umfassen. In dieser Differenzkarte zwischen der ersten Tiefenkarte und der zweiten Tiefenkarte kann dann die Lokalspule isoliert werden. Ein mögliches Vorgehen dafür ist in der folgenden Ausführungsform beschrieben.

Eine Ausführungsform sieht vor, dass das Ermitteln der Position der Lokalspule ein Anpassen eines geometrischen Modells der Lokalspule an die Abweichungen zwischen der ersten Tiefenkarte und der zweiten Tiefenkarte umfasst.

Wird eine Differenzkarte zwischen der ersten Tiefenkarte und der zweiten Tiefenkarte gebildet, so kann das geometrische Modell der Lokalspule an die Differenz-Pixelwolke, welche sich an der Position der Lokalspule in der Differenzkarte ergibt, angepasst werden. Dafür können Merkmale der Differenz-Pixelwolke, wie beispielsweise ein Schwerpunkt und/oder Hauptachsen, zum Anpassen des geometrischen Modells der Lokalspule ermittelt werden. Auch kann eine Vorverarbeitung der Differenzkarte mittels verschiedener Verfahren erfolgen, beispielsweise mittels einer Schwellwertoperation, einer Clusteringoperation, einem Region-Growing-Verfahren, einer morphologischen Dilatation bzw. Erosion, usw.

Das geometrische Modell kann abhängig von einer Art und/oder einem Typ der verwendeten Lokalspule ausgewählt werden. Die Art und/oder der Typ der verwendeten Lokalspule kann aus Spulenfiles ausgelesen werden, welche sich anhand der Steckung der Lokalspule ergeben. Das geometrische Modell wird für das Erkennen der Position der Lokalspule insbesondere aus einer Datenbank geladen. Das geometrische Modell der Lokalspule kann ein dreidimensionales CAD Modell oder Drahtgittermodell sein und beispielsweise auf Fertigungsdaten oder Bilddaten der Lokalspule basieren. Die Verwendung des Drahtgittermodells kann eine Berücksichtigung von strukturellen Informationen über die Lokalspule, welche den Raumpositionen zugeordnet werden können, ermöglichen. In alternativen Fällen kann das geometrische Modell der Lokalspule auch eine reine Punktwolke oder Vertizes (Ebenen durch drei Punkte inkl. einer Normale zur Ebene) umfassen.

Das Anpassen des geometrischen Modells der Lokalspule an die Abweichungen zwischen der ersten Tiefenkarte und der zweiten Tiefenkarte kann mittels eines dem Fachmann als sinnvoll erscheinenden Optimierungsverfahren, welches eine Minimierung eines Abstandsmaßes einsetzt, erfolgen. Beispielsweise kann ein Point-Matching-Verfahren oder iteratives Optimierungsverfahren eingesetzt werden. Das geometrische Modell der Lokalspule kann mittels einer Minimierung eines Abstandsmaßes zwischen dem geometrischen Modell und den Abweichungen zwischen der ersten Tiefenkarte und der zweiten Tiefenkarte angepasst werden. Bei der Anpassung des geometrischen Modells können auch weitere Eigenschaften der Lokalspule, beispielsweise eine Flexibilität der Lokalspule und/oder Randbedingungen, welche sich aus der Form der Lokalspule ergeben, berücksichtigt werden.

Insgesamt kann ein solches Vorgehen eine besonders robuste Erkennung der Position der Lokalspule anhand der akquirierten Tiefenkarten ermöglichen. Insbesondere aufgrund einer möglichen Einschränkung eines Suchbereichs kann ein solches Vorgehen auch besonders performant die Position der Lokalspule erkennen.

Eine Ausführungsform sieht vor, dass ein Ermitteln der Positionen von mehreren Lokalspulen, welche zum Erfassen der Magnetresonanz-Signale während der Magnetresonanz-Bildgebung des Untersuchungsobjekts vorgesehen sind, anhand der Tiefenkarte erfolgt, wobei für das Ermitteln der Positionen der mehreren Lokalspulen unterschiedliche Marker, welche und auf einer Oberfläche der mehreren Lokalspulen angebracht sind, in einem mittels der Tiefenkamera akquirierten Bild erkannt werden.

Die Marker sind dabei vorteilhafterweise optische Marker, welche in einem herkömmlichen optischen Bild, welches mittels der Tiefenkamera akquiriert wird, erkannt werden können. Die Marker sind insbesondere so ausgebildet, dass sie in dem mittels der Tiefenkamera akquirierten Bild unterschiedlich erscheinen. Beispielsweise können die Marker unterschiedliche Farben aufweisen. Eine besonders vorteilhafte Möglichkeit ist, dass die unterschiedlichen Marker unterschiedlich stark Infrarotlicht reflektieren, wobei die unterschiedlichen Marker in einem mittels der Tiefenkamera akquirierten Infrarot-bild erkannt werden. Dieses Vorgehen kann es erleichtern bzw. ermöglichen, eine Trennung der mehreren Lokalspulen in der Tiefenkarte, beispielsweise in der Differenz-Pixelwolke zwischen der ersten Tiefenkarte und der zweiten Tiefenkarte, durchzuführen. So kann die Verwendung der unterschiedlichen Marker beispielsweise eine Zuordnung einer Position und/oder Geometrie einer Lokalspule der mehreren Lokalspulen zu der passenden Spulenidentifikation im System ermöglichen.

Eine Ausführungsform sieht vor, dass das Ermitteln der Position der Lokalspule unter Verwendung eines Körpermodells erfolgt, welches an das Untersuchungsobjekt angepasst wird, wobei zum Ermitteln der Position der Lokalspule Abweichungen zwischen der Tiefenkarte und dem an das Untersuchungsobjekt angepassten Körpermodell bestimmt werden.

Das Körpermodell kann dabei unter Verwendung einer weiteren Tiefenkarte, welche vor der Positionierung der Lokalspule vom Untersuchungsobjekt erfasst wird, an das Untersuchungsobjekt angepasst werden. Es ist allerdings besonders vorteilhaft, dass das Körpermodell unter Verwendung einer Tiefenkarte, welche nach der Positionierung der Lokalspule vom Untersuchungsobjekt erfasst wird, an das Untersuchungsobjekt angepasst wird. Dann wird das Körpermodell vorteilhafterweise anhand von Landmarken, welche nicht von der auf dem Untersuchungsobjekt positionierten Lokalspule verdeckt werden, an das Untersuchungsobjekt angepasst.

In dem in dieser Ausführungsform beschriebenen Fall werden also die Abweichungen nicht zwischen zwei Tiefenkarten bestimmt. Vielmehr kann das an das Untersuchungsobjekt angepasste Körpermodell eine Referenz-Körperoberfläche bereitstellen, von welcher sich die in der Tiefenkarte erfasste Oberfläche der Lokalspule abhebt. Die Abweichungen zwischen der Position der Körperoberfläche des an das Untersuchungsobjekt angepassten Körpermodells und der Oberfläche der Lokalspule können somit zum Ermitteln der Position der Lokalspule verwendet werden. Gleichermaßen kann wiederum ein Modell der Lokalspule an die Abweichungen, analog zum bereits beschriebenen Vorgehen, zum Bestimmen der Position der Lokalspule angepasst werden. Vorteilhafterweise kann gemäß dieser Ausführungsform die Position der Lokalspule unter Verwendung lediglich einer akquirierten Tiefenkarte bestimmt werden.

Eine Ausführungsform sieht vor, dass das Ermitteln der Vorbereitungsinformation einen Vergleich der ermittelten Position der Lokalspule mit einer gewünschten Position für die Lokalspule zum Empfangen von Magnetresonanz-Signalen aus dem zu untersuchenden Organ umfasst, wobei die gewünschte Position für die Lokalspule anhand der berechneten Lokalisierungswahrscheinlichkeit des zu untersuchenden Organs ermittelt wird.

Derart wird die Lokalisierungswahrscheinlichkeit des in der Magnetresonanz-Bildgebung zu untersuchenden Organs in einem Körper des Untersuchungsobjekts unter Verwendung der Tiefenkarte berechnet. Weiterhin wird ebenfalls unter Verwendung der Tiefenkarte, insbesondere unter Verwendung der besagten ersten Tiefenkarte und zweiten Tiefenkarte, die Position der Lokalspule, welche zum Empfangen von Magnetresonanz-Signalen während der Magnetresonanz-Bildgebung des Untersuchungsobjekts vorgesehen ist, ermittelt. Derart ist der Vergleich der ermittelten Position der Lokalspule mit der gewünschten Position für die Lokalspule, welche anhand der berechneten Lokalisierungswahrscheinlichkeit des zu untersuchenden Organs ermittelt wird, möglich. Derart gehen als Eingangsparameter in das Ermitteln der Vorbereitungsinformation sowohl die berechneten Lokalisierungswahrscheinlichkeit des zu untersuchenden Organs als auch die ermittelte Position der Lokalspule ein.

Die gewünschte Position der Lokalspule wird insbesondere anhand einer höchsten Lokalisierungswahrscheinlichkeit des zu untersuchenden Organs im Körper des Untersuchungsobjekts ermittelt. Die gewünschte Position der Lokalspule kann derart ausgebildet sein, dass ein Zentrum der Lokalspule auf der Körperoberfläche des Untersuchungsobjekts möglichst senkrecht direkt über der Position im Körper des Untersuchungsobjekts positioniert ist, an welchem die höchste Lokalisierungswahrscheinlichkeit des zu untersuchenden Organs ermittelt worden ist. Die gewünschte Position der Lokalspule kann derart berechnet werden, dass die Lokalspule bei einer Positionierung an der gewünschten Position besonders geeignet bei der Magnetresonanz-Bildgebung Magnetresonanz-Signale aus dem zu untersuchenden Organ empfangen kann. In das Ermitteln der gewünschten Position der Lokalspule kann zusätzlich zu der Lokalisierungswahrscheinlichkeit des zu untersuchenden Organs ein Empfangsprofil der Lokalspule berücksichtigt werden.

Der Vergleich zwischen der ermittelten Position der Lokalspule und der gewünschten Position der Lokalspule kann ein Ermitteln eines Grads der Übereinstimmung zwischen der ermittelten Position der Lokalspule und der gewünschten Position der Lokalspule umfassen. Alternativ oder zusätzlich kann der Vergleich zwischen der ermittelten Position der Lokalspule und der gewünschten Position der Lokalspule ein Ermitteln einer Richtung, insbesondere einer Translationsrichtung und/oder eines Rotationswinkels, umfassen, in welcher die Lokalspule verschoben und/oder rotiert werden muss, so dass die Lokalspule von der ermittelten Position an die gewünschte Position umgelagert wird. Ergebnisse aus dem Vergleich zwischen der ermittelten Position der Lokalspule und der gewünschten Position der Lokalspule können als Vorbereitungsinformation geeignet bereitgestellt, beispielsweise auf einer Anzeigeeinheit ausgegeben, werden.

Gegenüber bekannten Sensoren zur Detektion der Position der Lokalspule, wie beispielsweise B0-Hallsensoren, Ultraschallsensoren, RFID Tags, können mittels der Tiefenkamera gleichzeitig die Lokalisierungswahrscheinlichkeit des Organs berechnet werden und die Position der Lokalspule erkannt werden. Derart können beide Erkennungsschritte vorheilhafterweise mit dem gleichen Sensor, nämlich der Tiefenkamera, durchgeführt werden. Ein Synergieeffekt durch die zweimalige Verwendung der Tiefenkarte für unterschiedliche Erkennungsschritte ist demnach gegeben. Derart bietet das Erfassen der Tiefenkarte eine besonders vorteilhafte Grundlage zum Ermitteln der Vorbereitungsinformation basierend auf der Lokalisierungswahrscheinlichkeit des zu untersuchenden Organs und der ermittelten Position der Lokalspule.

Der Vergleich der ermittelten Position der Lokalspule mit der gewünschten Position für die Lokalspule kann eine Berücksichtigung eines Empfangsprofils der Lokalspule umfassen, wobei das Empfangsprofil der Lokalspule auf Grundlage des geometrischen Modells der Lokalspule ermittelt wird. Dafür ist insbesondere zu dem geometrischen Modell der Lokalspule eine Information über das Empfangsprofil der Lokalspule hinterlegt. Das Empfangsprofil der Lokalspule kann in Bezug auf das Drahtgittermodell der Lokalspule hinterlegt sein. Bei der Anpassung des geometrischen Modells der Lokalspule an die Abweichungen zwischen der ersten Tiefenkarte und der zweiten Tiefenkarte kann auch die Information über das Empfangsprofil der Lokalspule an die aktuellen Gegebenheiten angepasst werden. Derart kann besonders geeignet das Empfangsprofil der Lokalspule bei dem Ermitteln der gewünschten Position der Lokalspule berücksichtigt werden.

Das Empfangsprofil der Lokalspule kann auch mittels eines an das Untersuchungsobjekt angepassten Körpermodells simuliert werden. Dabei kann das an das Untersuchungsobjekt angepasste Körpermodell Informationen über eine Lokalisierung von Organen und den zugehörigen Gewebeeigenschaften bereitstellen. Diese Informationen können zur Simulation des tatsächlich bei der Untersuchung vorliegenden Empfangsprofils der Lokalspule verwendet werden.

Eine Ausführungsform sieht vor, dass das Bereitstellen der Vorbereitungsinformation eine Ausgabe eines Vorschlags zur Repositionierung der Lokalspule basierend auf einem Ergebnis des Vergleichs umfasst.

Die Ausgabe des Vorschlags zur Repositionierung erfolgt insbesondere auf einer Anzeigeeinheit, welche auch als Touch-Interface, ausgebildet werden kann. Die Ausgabe des Vorschlags zur Repositionierung kann auch mittels der bereits beschriebenen Projektionseinheit erfolgen. Weitere Ausgabemöglichkeiten zur Ausgabe des Vorschlags zur Repositionierung der Lokalspule, beispielsweise eine Ausgabe mittels einer haptischen Einheit oder einer Audioausgabeeinheit, sind ebenfalls denkbar.

Ist das Ergebnis des Vergleichs zwischen der ermittelten Position der Lokalspule und der gewünschten Position der Lokalspule, dass sich die Lokalspule bereits an der gewünschten Position befindet, so kann ausgegeben werden, dass keine Repositionierung der Lokalspule mehr nötig ist bzw. dass sich die Lokalspule an der gewünschten Position befindet. Ansonsten kann eine Verfahrensanweisung an das Bedienpersonal zum Umsetzen des Vorschlags zur Repositionierung der Lokalspule ausgegeben werden. Beispielsweise kann ein Verschiebungsvektor, beispielsweise mittels der Projektionseinheit oder auf der Anzeigeeinheit, angezeigt werden, um die Lokalspule von der aktuellen ermittelten Position an die gewünschte Position korrekt verschieben zu können. Auch kann ein Vorschlag für eine noch benötigte Rotation und/oder Verformung der Lokalspule ausgegeben werden. Der Vorschlag zur Repositionierung der Lokalspule kann auch an eine Weiterverarbeitungseinheit ausgegeben werden, welche automatisch oder semiautomatisch die Repositionierung der Lokalspule steuern kann.

Insgesamt bietet der Vorschlag zur Repositionierung der Lokalspule eine besonders geeignete Möglichkeit, um die korrekte Position der Lokalspule zum Empfangen der Magnetresonanz-Signale aus dem zu untersuchenden Organ sicherzustellen. Gerade bei einem Vorliegen einer nicht sichtbaren Anatomie des Bedienobjekts oder wenn unerfahrenes Bedienpersonal die Vorbereitung der Magnetresonanz-Bildgebung durchführt, kann diese Vorbereitungsinformation zu einer deutlichen Erhöhung der Qualität der Vorbereitung der Magnetresonanz-Bildgebung führen.

Das erfindungsgemäße System umfasst ein Magnetresonanzgerät mit einer Patientenlagerungsvorrichtung, eine Tiefenkamera, eine Bereitstellungseinheit und eine Recheneinheit, wobei das System zum Ausführen eines erfindungsgemäßen Verfahrens ist. Derart ist das erfindungsgemäße System zum Ausführen eines Verfahrens zum Bereitstellen einer Vorbereitungsinformation zur Vorbereitung einer Magnetresonanz-Bildgebung eines Untersuchungsobjekts mittels eines Magnetresonanzgeräts ausgebildet.

Dabei kann die Recheneinheit zum Steuern von anderen Komponenten des Systems ausgebildet sein. Auch ist insbesondere die Recheneinheit dazu ausgebildet, computerlesbare Instruktionen auszuführen, um das erfindungsgemäße Verfahren auszuführen bzw. die Steuerung der Ausführung des erfindungsgemäßen Verfahrens vorzunehmen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen, um ein erfindungsgemäßes bzw. die Steuerung der Ausführung des erfindungsgemäßen Verfahren auszuführen.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das System ist dazu ausgebildet, dass in einem ersten Schritt eine Lagerung des Untersuchungsobjekts auf einer Patientenlagerungsvorrichtung des Magnetresonanzgeräts erfolgen kann. Die Tiefenkamera ist anschließend zum Erfassen einer Tiefenkarte des auf der Patientenlagerungsvorrichtung gelagerten Untersuchungsobjekts ausgebildet. Die Recheneinheit kann eine Vorbereitungsinformation zur Vorbereitung der Magnetresonanz-Bildgebung unter Verwendung der erfassten Tiefenkarte ermitteln. Die Bereitstellungseinheit kann schließlich die Vorbereitungsinformation bereitstellen.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile der erfindungsgemäßen Systems und des erfindungsgemäßen Computerprogrammprodukts entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
- Fig. 1: ein erfindungsgemäßes System,
- Fig. 2: ein Ablaufdiagramm eines Verfahrens zum Bereitstellen einer Vorbereitungsinformation,
- Fig. 3: ein Ablaufdiagramm einer Ausführungsform des erfindungsgemäßen Verfahrens,
- Fig. 4: ein Ablaufdiagramm einer Ausführungsform des erfindungsgemäßen Verfahrens,
- Fig. 5: ein Ablaufdiagramm einer Ausführungsform des erfindungsgemäßen Verfahrens,
- Fig. 6: ein mögliches Körpermodell zur Anpassung an das Untersuchungsobjekt unter Verwendung der Tiefenkarte,
- Fig. 7: eine mögliche Positionierung von Landmarken für die Anpassung des Körpermodells an das Untersuchungsobjekt,
- Fig. 8: exemplarische Abweichungen zwischen einer ersten Tiefenkarte und einer zweiten Tiefenkarte in einer Schnittdarstellung,
- Fig. 9: exemplarische Abweichungen zwischen einer ersten Tiefenkarte und einer zweiten Tiefenkarte in einer Draufsichtdarstellung,
- Fig. 10: ein mögliches geometrisches Modell einer Lokalspule zum Anpassen an die Abweichungen zwischen der ersten Tiefenkarte und der zweiten Tiefenkarte und
- Fig. 11: eine exemplarische Ausgabe eines Vorschlags zur Repositionierung einer Lokalspule.

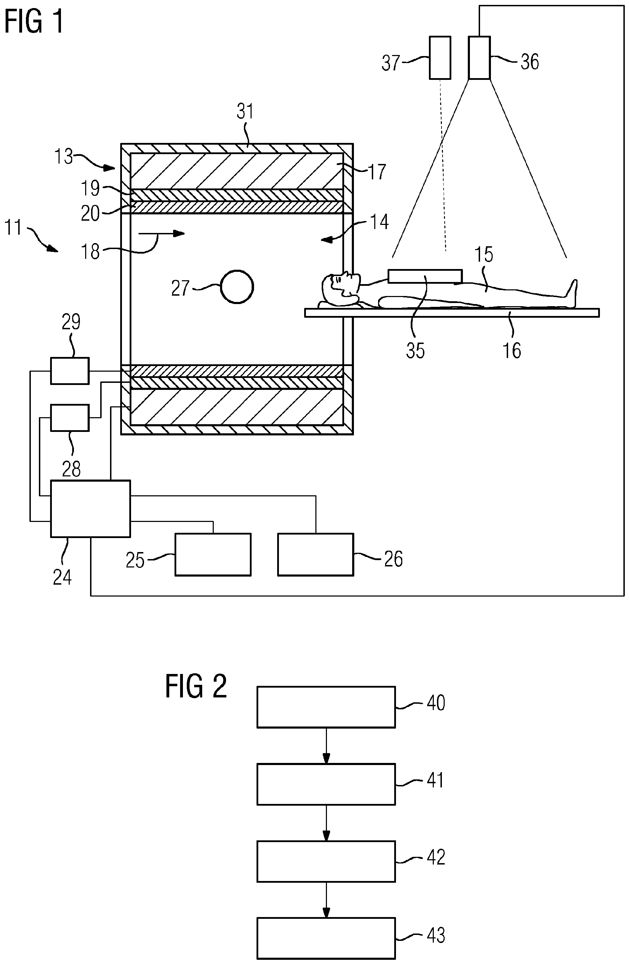

**Fig. 1** stellt ein erfindungsgemäßes System schematisch dar.

Das System umfasst ein Magnetresonanzgerät 11. Das Magnetresonanzgerät 11 umfasst eine von einer Magneteinheit 13 gebildete Detektoreinheit mit einem Hauptmagneten 17 zu einem Erzeugen eines starken und insbesondere konstanten Hauptmagnetfelds 18. Das Hauptmagnetfeld 18 weist einen, insbesondere annähernd kugelförmigen, Bereich größer Homogenität auf, welcher als Isozentrum 27 bezeichnet werden kann. Zudem weist das Magnetresonanzgerät 11 einen zylinderförmigen Patientenaufnahmebereich 14 zu einer Aufnahme eines Untersuchungsobjekts 15, im vorliegenden Fall eines Patienten, auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14, insbesondere in das Isozentrum 27, geschoben werden. Die Patientenlagerungsvorrichtung 16 weist hierzu einen Liegentisch auf, der bewegbar innerhalb des Magnetresonanzgeräts 11 angeordnet ist. Die Magneteinheit 13 ist mittels einer Gehäuseverkleidung 31 des Magnetresonanzgeräts nach außen hin abgeschirmt.

Die Magneteinheit 13 weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 11 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Magnetresonanz-Sequenzen in einen Untersuchungsraum, der im Wesentlichen von dem Patientenaufnahmebereich 14 gebildet ist, ein. Die Hochfrequenzantenneneinheit 20 ist weiterhin zum Empfang von Magnetresonanz-Signalen, insbesondere aus dem Patienten 15, ausgebildet. Weiterhin umfasst das Magnetresonanzgerät 11 umfasst im in Fig. 1 gezeigten Fall eine Lokalspule 35, welche auf einer zu untersuchenden Körperregion des Patienten 15 zum Empfangen von Magnetresonanz-Signalen positioniert ist.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

Weiterhin weist das System eine Recheneinheit 24 auf. Die Recheneinheit 24 kann das Magnetresonanzgerät 11 steuern. Die Recheneinheit kann auch mit einer Anzeigeeinheit 25 und einer Eingabeeinheit 26 in Verbindung stehen. Vorbereitungsinformationen zu der Vorbereitung der Magnetresonanz-Bildgebung können auf der Anzeigeeinheit 25 einen Benutzer bereitgestellt werden. Mittels der Eingabeeinheit 26 können Informationen und/oder Parameter für die Vorbereitung der Magnetresonanz-Bildgebung eingegeben werden können. Auch können die Anzeigeeinheit 25 und die Eingabeeinheit 26 als kombiniertes Touch-Interface ausgebildet sein. Auch kann eine in Fig. 1 dargestellte Projektionseinheit 37 zur Projektion von Projektionsdaten auf eine Körperoberfläche des Patienten 15 bzw. auf die Lokalspule 35 ausgebildet sein.

Das System umfasst weiterhin eine Tiefenkamera 36, welche zum Erfassen einer Tiefenkarte des Patienten 15 bzw. der Lokalspule 35 ausgebildet ist. Die Tiefenkamera 36 ist in dem Untersuchungsraum, in welchem sich auch das Magnetresonanzgerät 11 befindet, vorteilhafterweise an der Decke des Untersuchungsraums, positioniert.

Das System ist zusammen mit dem Magnetresonanzgerät 11, der Recheneinheit 24 und der Tiefenkamera 36 zur Ausführung eines erfindungsgemäßen Verfahrens zum Bereitstellen einer Vorbereitungsinformation ausgelegt.

**Fig. 2** zeigt ein Ablaufdiagramm eines Verfahrens zum Bereitstellen einer Vorbereitungsinformation zur Vorbereitung einer Magnetresonanz-Bildgebung eines Untersuchungsobjekts 15 mittels eines Magnetresonanzgeräts 11 Die Schritte dieses Verfahrens finden Anwendung im erfindungsgemässen Verfahren, welches weiter unten beschrieben wird.

In einem ersten Verfahrensschritt 40 erfolgt eine Lagerung des Untersuchungsobjekts 15 auf der Patientenlagerungsvorrichtung 16 des Magnetresonanzgeräts 11.

In einem weiteren Verfahrensschritt 41 erfolgt ein Erfassen einer Tiefenkarte des auf der Patientenlagerungsvorrichtung 16 gelagerten Untersuchungsobjekts 15 mittels der Tiefenkamera 36.

In einem weiteren Verfahrensschritt 42 erfolgt ein Ermitteln einer Vorbereitungsinformation zur Vorbereitung der Magnetresonanz-Bildgebung unter Verwendung der erfassten Tiefenkarte mittels der Recheneinheit 24.

In einem weiteren Verfahrensschritt 43 erfolgt ein Bereitstellen der Vorbereitungsinformation mittels der Recheneinheit 24 und/oder der Anzeigeeinheit 25 und/oder der Projektionseinheit 37.

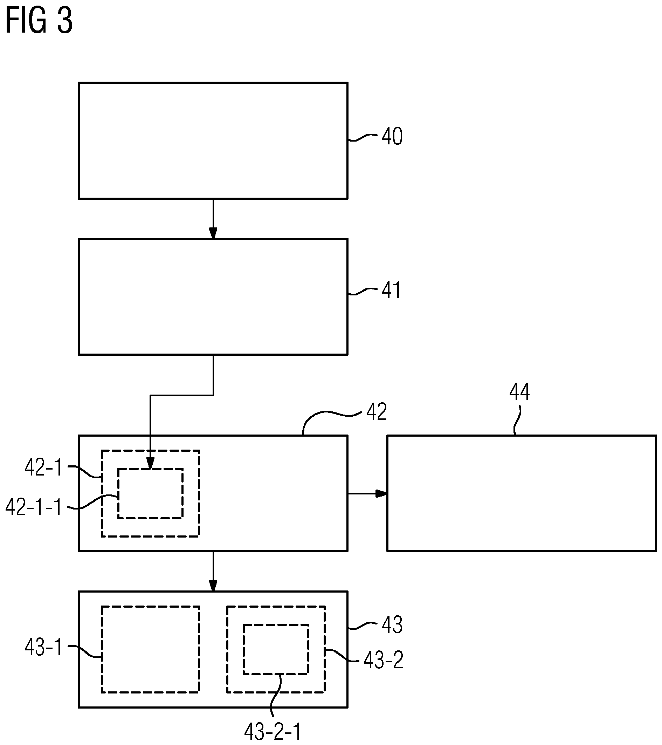

Die nachfolgenden Beschreibungen der Ausführungsformen in Fig. 3 bis Fig. 5 beschränken sich im Wesentlichen auf die Unterschiede zu dem Verfahren in Fig. 2, wobei bezüglich gleich bleibender Verfahrensschritte auf die Beschreibung der Fig. 2 verwiesen wird. Im Wesentlichen gleich bleibende Verfahrensschritte sind grundsätzlich mit den gleichen Bezugszeichen beziffert.

Die in Fig. 3 bis Fig. 5 gezeigten Ausführungsformen des erfindungsgemäßen Verfahrens umfasst im Wesentlichen die Verfahrensschritte 40, 41, 42, 43 des Verfahrens gemäß Fig. 2. Zusätzlich umfassen die in Fig. 3 bis Fig. 5 gezeigte Ausführungsform des erfindungsgemäßen Verfahrens zusätzliche Verfahrensschritte und Unterschritte. Denkbar ist auch ein zu Fig. 3 bis Fig. 5 alternativer Verfahrensablauf, welcher nur einen Teil der in Fig. 3 bis Fig. 5 dargestellten zusätzlichen Verfahrensschritte und/oder Unterschritte aufweist. Selbstverständlich kann auch ein zu Fig. 3 bis Fig. 5 alternativer Verfahrensablauf zusätzliche Verfahrensschritte und/oder Unterschritte aufweisen.

**Fig. 3** zeigt ein Ablaufdiagramm eines erfindungsgemäßen Verfahrens zum Bereitstellen einer Vorbereitungsinformation zur Vorbereitung einer Magnetresonanz-Bildgebung eines Untersuchungsobjekts 15 mittels eines Magnetresonanzgeräts 11.

Im in Fig. 3 gezeigten Fall umfasst das Ermitteln der Vorbereitungsinformation einen Teilschritt 42-1, in welchem ein Berechnen einer Lokalisierungswahrscheinlichkeit eines in der Magnetresonanz-Bildgebung zu untersuchenden Organs in einem Körper des Untersuchungsobjekts unter Verwendung der Tiefenkarte erfolgt. Dieser Teilschritt 42-1 umfasst erfindungsgemäss einen weiteren Teilschritt 42-1-1, in welchem das Berechnen der Lokalisierungswahrscheinlichkeit des zu untersuchenden Organs eine Anpassung eines Körpermodells, welches eine Information über eine Lokalisierung des zu untersuchenden Organs im Körpermodell umfasst, an das Untersuchungsobjekt unter Verwendung der Tiefenkarte umfasst.

Für das das Bereitstellen der Vorbereitungsinformation im weiteren Verfahrensschritt 43 gibt es nun zwei Möglichkeiten, welche in zwei unterschiedlichen Teilschritten 43-1, 43-2 realisiert sein können. Die beiden unterschiedlichen Teilschritte 43-1, 43-2 können dabei unabhängig zueinander oder kombiniert durchgeführt werden.

In einem ersten Teilschritt 43-1 kann das Bereitstellen der Vorbereitungsinformation eine Anzeige von Anzeigedaten, welche basierend auf der Lokalisierungswahrscheinlichkeit des zu untersuchenden Organs erstellt werden, auf einer Anzeigeeinheit zusammen mit einer Darstellung einer Repräsentation eines menschlichen Körpers umfassen. In einem zweiten Teilschritt 43-2 kann das Bereitstellen der Lokalisierungswahrscheinlichkeit eine Projektion von Projektionsdaten, welche basierend auf der Lokalisierungswahrscheinlichkeit des zu untersuchenden Organs erstellt werden, auf eine Körperoberfläche des Untersuchungsobjekts mittels einer Projektionseinheit umfassen. Dabei kann in einem weiteren Teilschritt 43-2-1 als Projektionsdaten zumindest ein Konfidenzradius auf die Körperoberfläche des Untersuchungsobjekts projiziert werden, wobei der zumindest eine Konfidenzradius die Lokalisierungswahrscheinlich des zu untersuchenden Organs wiederspiegelt. Anhand der ermittelten Lokalisierungswahrscheinlichkeit kann gemäß Fig. 3 die Patientenlagerungsvorrichtung des Magnetresonanzgeräts in einem weiteren Verfahrensschritt 44 derart verfahren werden, dass für die Magnetresonanz-Bildgebung das zu untersuchende Organ automatisch in einem Isozentrum des Magnetresonanzgeräts positioniert wird.

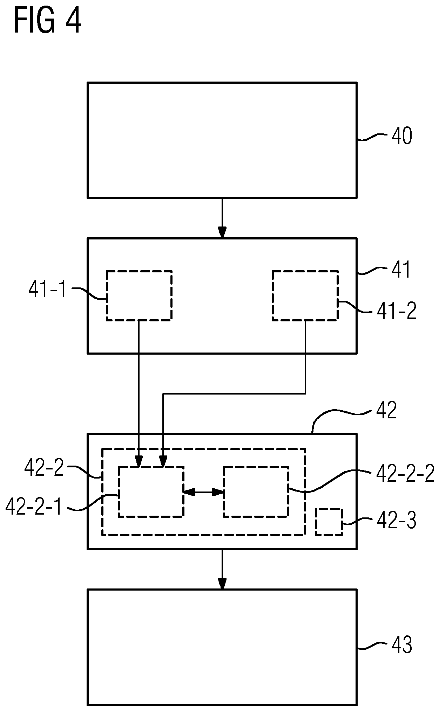

**Fig. 4** zeigt ein Ablaufdiagramm einer Ausführungsform eines erfindungsgemäßen Verfahrens zum Bereitstellen einer Vorbereitungsinformation zur Vorbereitung einer Magnetresonanz-Bildgebung eines Untersuchungsobjekts 15 mittels eines Magnetresonanzgeräts 11.

Im in Fig. 4 gezeigten Fall bildet die im Verfahrensschritt 41 erfasste Tiefenkarte eine Lokalspule ab, welche zum Empfangen von Magnetresonanz-Signalen während der Magnetresonanz-Bildgebung des Untersuchungsobjekts vorgesehen ist, Derart umfasst das Ermitteln der Vorbereitungsinformation in einem Teilschritt 42-2 des weiteren Verfahrensschritts 42 ein Ermitteln einer Position der Lokalspule anhand der Tiefenkarte.

Dafür umfasst das Erfassen der Tiefenkarte in einem ersten Teilschritt 41-1 des weiteren Verfahrensschritts 41 ein Erfassen einer ersten Tiefenkarte zeitlich vor einer Positionierung der Lokalspule für die Magnetresonanz-Bildgebung und in einem zweiten Teilschritt 41-2 des weiteren Verfahrensschritts 41 ein Erfassen einer zweiten Tiefenkarte zeitlich nach der Positionierung der Lokalspule für die Magnetresonanz-Bildgebung. Das Ermitteln der Position der Lokalspule umfasst dann in einem Unterschritt 42-2-1 des Teilschritts 42-2 eine Bestimmung von Abweichungen zwischen der ersten Tiefenkarte und der zweiten Tiefenkarte. Hierbei kann das Ermitteln der Position der Lokalspule in einem Unterschritt 42-2-2 des Teilschritts 42-2 ein Anpassen eines geometrischen Modells der Lokalspule an die Abweichungen zwischen der ersten Tiefenkarte und der zweiten Tiefenkarte umfassen.

Im weiteren Verfahrensschritt 42 kann auch in einem weiteren Teilschritt 42-3 die Position einer weiteren Lokalspule bestimmt werden. Wenn ein Ermitteln der Positionen von mehreren Lokalspulen, welche zum Erfassen der Magnetresonanz-Signale während der Magnetresonanz-Bildgebung des Untersuchungsobjekts vorgesehen sind, anhand der Tiefenkarte erfolgt, ist es sinnvoll, dass für das Ermitteln der Positionen der mehreren Lokalspulen unterschiedliche Marker, welche und auf einer Oberfläche der mehreren Lokalspulen angebracht sind, in einem mittels der Tiefenkamera akquirierten Bild erkannt werden. Alternativ ist es auch denkbar, dass das Ermitteln der Position der Lokalspule unter Verwendung eines Körpermodells erfolgt, welches an das Untersuchungsobjekt angepasst wird, wobei zum Ermitteln der Position der Lokalspule Abweichungen zwischen der Tiefenkarte und dem an das Untersuchungsobjekt angepassten Körpermodell bestimmt werden. Dann ist es nicht notwendig, zwei Tiefenkarten vom Untersuchungsobjekt zum Bestimmen der Position der Lokalspule zu erfassen.

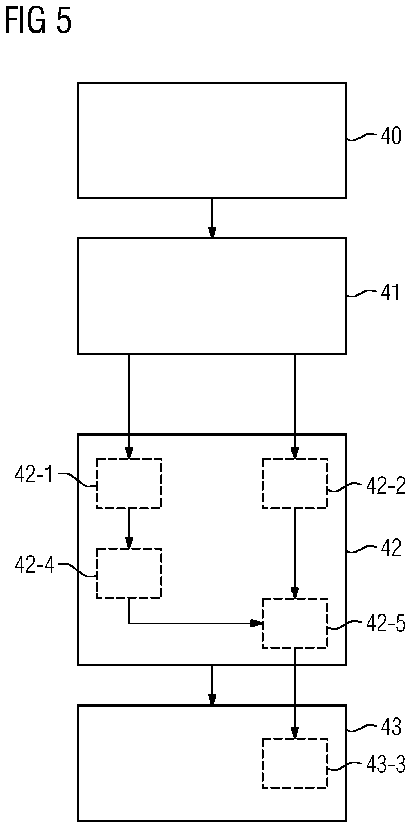

**Fig. 5** zeigt ein Ablaufdiagramm einer Ausführungsform eines erfindungsgemäßen Verfahrens zum Bereitstellen einer Vorbereitungsinformation zur Vorbereitung einer Magnetresonanz-Bildgebung eines Untersuchungsobjekts 15 mittels eines Magnetresonanzgeräts 11.

Gemäss Fig. 5 erfolgen der Teilschritt 42-1 des weiteren Verfahrensschritts 42 gemäß Fig. 2 und der Teilschritt 42-2 des weiteren Verfahrensschritts gemäß Fig. 3. Das Ermitteln der Vorbereitungsinformation umfasst also das Berechnen der Lokalisierungswahrscheinlichkeit des zu untersuchenden Organs und das Ermitteln der Position der Lokalspule.

In einem weiteren Teilschritt 42-4 des weiteren Verfahrensschritts 42 wird nun eine gewünschte Position für die Lokalspule anhand der berechneten Lokalisierungswahrscheinlichkeit des zu untersuchenden Organs ermittelt. Das Ermitteln der Vorbereitungsinformation umfasst einen Vergleich der ermittelten Position der Lokalspule mit einer gewünschten Position für die Lokalspule zum Empfangen von Magnetresonanz-Signalen aus dem zu untersuchenden Organ in einem weiteren Teilschritt 42-5 des weiteren Verfahrensschritts 42.

Das Bereitstellen der Vorbereitungsinformation im weiteren Verfahrensschritt 43 kann dann eine Ausgabe eines Vorschlags zur Repositionierung der Lokalspule basierend auf einem Ergebnis des Vergleichs in einem Teilschritt 43-3 des weiteren Verfahrensschritts 43 umfassen.

Die in Fig. 2-5 dargestellten Verfahrensschritte des erfindungsgemäßen Verfahrens werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das erfindungsgemäße Verfahren auszuführen, wenn das Computerprogramm und/oder die Software in der Recheneinheit mittels einer Prozessoreinheit der Recheneinheit ausgeführt wird.

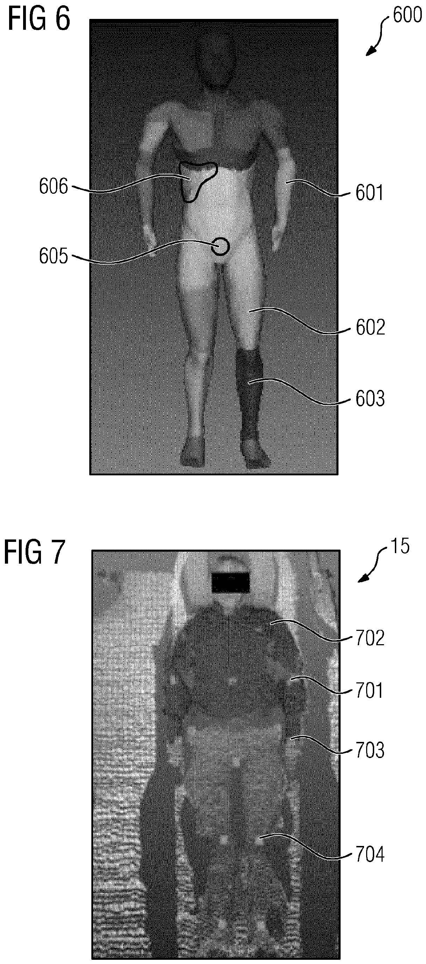

**Fig. 6** zeigt ein mögliches Körpermodell 600 zur Anpassung an das Untersuchungsobjekt 15 unter Verwendung der Tiefenkarte. Das in Fig. 6 gezeigte Körpermodell 600 kann demnach im Teilschritt 42-1-1 der Fig. 3 zum Berechnen der Lokalisierungswahrscheinlichkeit des zu untersuchenden Organs verwendet werden.

Im in Fig. 6 gezeigten Fall ist das Körpermodell 600 als dreidimensionales Standard-Modell eines menschlichen Körpers ausgebildet. Das Körpermodell 600 umfasst Konturinformationen des menschlichen Körpers, welche derart besonders einfach an die Tiefenkarte, welche vom Untersuchungsobjekt 15 akquiriert worden ist, angepasst werden kann. Für die Anpassung können Landmarken 701, 702, 703, 704 verwendet werden, beispielsweise wie sie in Fig. 7 dargestellt werden. Für die Anpassung umfasst das Körpermodell 600 vorteilhafterweise verschieden Körpersegmente 601, 602, 603, beispielsweise ein Armsegment 601, ein Oberschenkelsegment 602 und ein Unterschenkelsegment 603, welche unabhängig voneinander, insbesondere mittels der entsprechenden Landmarken 701, 702, 703, 704, an das Untersuchungsobjekt 15 angepasst werden können.

Zusätzlich zu den Konturinformationen umfasst das Körpermodell 600 eine Information über die Lokalisierung von zumindest einem Organ 605, 606, im gezeigten Fall beispielsweise der Prostata 605 und der Leber 604. Diese Information kann beispielsweise statistische Lokalisierungswahrscheinlichkeiten des zumindest einen Organs 605, 606 enthalten. Derart kann basierend auf der Anpassung des Körpermodells 600 an das Untersuchungsobjekt 15 die Information über die Lokalisierung des zu untersuchenden Organs an die patientenspezifischen Gegebenheiten der Lagerung des Untersuchungsobjekts 15 angepasst werden. Die Information über die Lokalisierung des zumindest einen Organs 605, 606 im Körpermodell 606 ist insbesondere an die Konturinformationen des Körpermodells 600, insbesondere die Körpersegmente 601, 602, 603, gekoppelt, so dass bei einer Anpassung der Konturinformationen des Körpermodells 600 an das Untersuchungsobjekt 15 auch die Information über die Lokalisierung des zumindest einen Organs 605, 606 an das Untersuchungsobjekt 15 angepasst wird.

**Fig. 7** zeigt eine mögliche Positionierung von Landmarken 701, 702, 703, 704 für die Anpassung des Körpermodells 600 an das Untersuchungsobjekt 15. Die Landmarken 701, 702, 703, 704 können im Körpermodell 600, beispielsweise einem Körpermodell 600 gemäß Fig. 6, definiert sein und im Untersuchungsobjekt 15 mittels eines geeigneten Detektionsalgorithmus oder manuell erkannt werden.

Die in Fig. 7 gezeigte Konfiguration der Landmarken 701, 702, 703, 704 ist selbstverständlich nur als ein vorteilhaftes Beispiel zu sehen. Definiert ist unter anderem eine erste Landmarke 701 an einem Ellenbogen des Untersuchungsobjekts 15, eine zweite Landmarke 702 an einer Schulter des Untersuchungsobjekts 15, eine dritte Landmarke 703 an einer Hand des Untersuchungsobjekts 15 und eine vierte Landmarke 704 an einem Knie des Untersuchungsobjekts 15. Selbstverständlich sind weitere nicht im Text erwähnte Landmarken definiert.

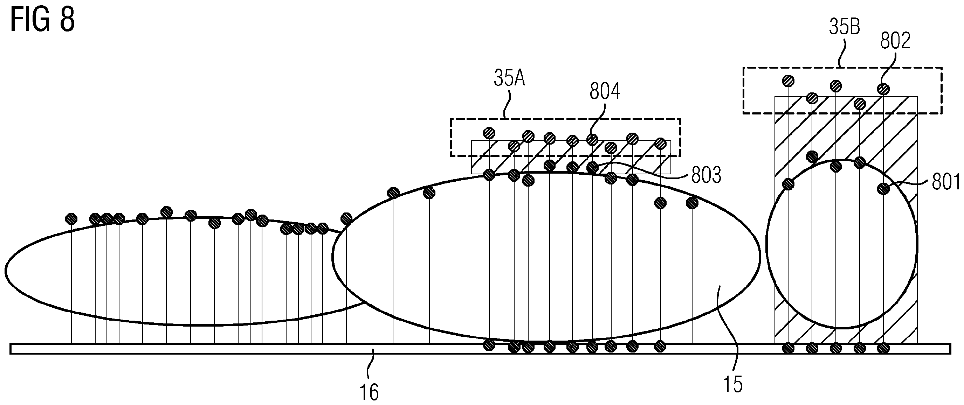

**Fig. 8** zeigt exemplarische Abweichungen zwischen einer ersten Tiefenkarte 801, 803 und einer zweiten Tiefenkarte 802, 804 in einer Schnittdarstellung.

In Fig. 8 ist ein sagittaler Schnitt durch das Untersuchungsobjekt 15, welches auf der Patientenlagerungsvorrichtung 16 positioniert ist, gezeigt. Auf dem Untersuchungsobjekt ist eine erste Lokalspule 35A, welche von einer flexiblen Oberflächenspule gebildet ist, und eine zweite Lokalspule 35B, welche von einer Kopfspule gebildet ist, positioniert.

Im ersten Teilschritt 41-1 des weiteren Verfahrensschritts 41 gemäß Fig. 4 ist eine erste Tiefenkarte 801, 803 zeitlich vor der Positionierung der Lokalspulen 35A, 35B für die Magnetresonanz-Bildgebung vom Untersuchungsobjekt 15 erfasst worden. Die erste Tiefenkarte 801, 803 bildet demnach lediglich die Körperoberfläche des Untersuchungsobjekts 15 ab. Die erste Tiefenkarte 801, 803 umfasst dabei mehrere Abtastpunkte, von denen in Fig. 8 ein Teil eingezeichnet ist und zwei exemplarisch mit Bezugszeichen beschriftet sind.

Im zweiten Teilschritt 41-2 des weiteren Verfahrensschritts 41 gemäß Fig. 4 ist eine zweite Tiefenkarte 802, 804 zeitlich nach der Positionierung der Lokalspulen 35A, 35B für die Magnetresonanz-Bildgebung vom Untersuchungsobjekt 15 erfasst worden. Die zweite Tiefenkarte 802, 804 umfasst demnach Tiefeninformationen der auf dem Untersuchungsobjekt 15 positionierten Lokalspulen 35A, 35B. Die zweite Tiefenkarte 802, 804 umfasst dabei mehrere Abtastpunkte, von denen in Fig. 8 ein Teil eingezeichnet ist und zwei exemplarisch mit Bezugszeichen beschriftet sind.

Es ist in Fig. 8 deutlich erkennbar, dass die zweite Tiefenkarte 802, 804 Tiefeninformationen umfasst, welche sich im Bereich der Lokalspulen 35A, 35B deutlich von den Tiefeninformationen der ersten Tiefenkarte 801, 803 unterscheiden. Derart kann eine Bestimmung der Abweichungen zwischen der ersten Tiefenkarte 801, 803 und der zweiten Tiefenkarte 802, 804 in einem Unterschritt 42-2-1 des Teilschritts 42-2 der Fig. 4 als Grundlage für eine Isolierung der Lokalspulen 35A, 35B dienen.

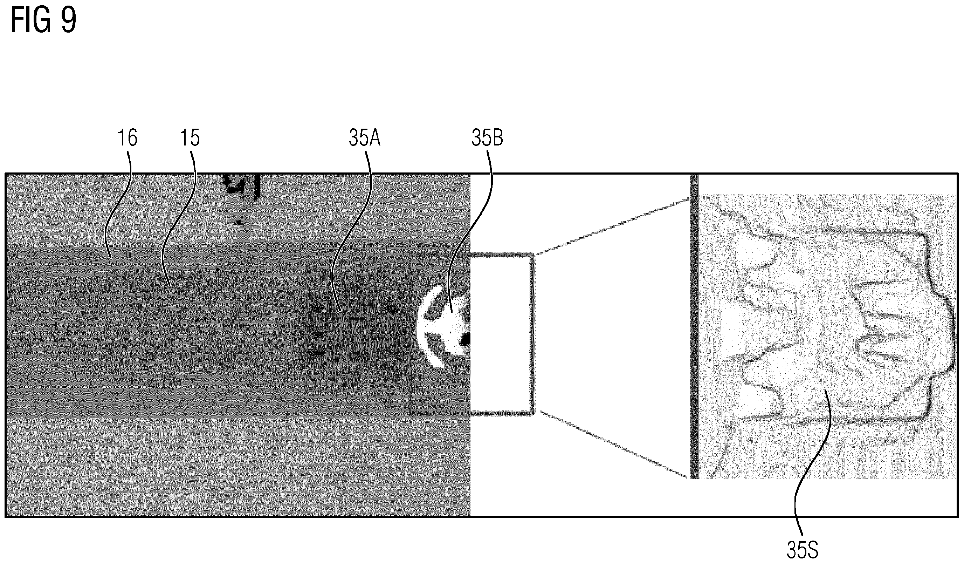

**Fig. 9** zeigt exemplarische Abweichungen zwischen einer ersten Tiefenkarte 801, 803 und einer zweiten Tiefenkarte 802, 804 in einer Draufsichtdarstellung.

In Fig. 9 ist dabei das gleiche Untersuchungssetup wie in Fig. 8 dargestellt. Fig. 9 zeigt eine Draufsicht auf in einem Unterschritt 42-2-1 des Teilschritts 42-2 der Fig. 4 bestimmte Abweichungen zwischen der ersten Tiefenkarte 801, 803 und der zweiten Tiefenkarte 802, 804.

In Fig. 9 sind die erste Lokalspule 35A und die zweite Lokalspule 35B hervorgehoben dargestellt, so wie sie in den Abweichungen zwischen der ersten Tiefenkarte 801, 803 und der zweiten Tiefenkarte 802, 804 erscheinen. Mittels geeigneter Operatoren, wie beispielsweise Schwellwertoperatoren und/oder Clusteringoperatoren, ist eine Vorsegmentierung der Lokalspulen 35A, 35B möglich. Eine segmentierte zweite Lokalspule 35S ist im rechten Bildausschnitt der Fig. 9 dargestellt.

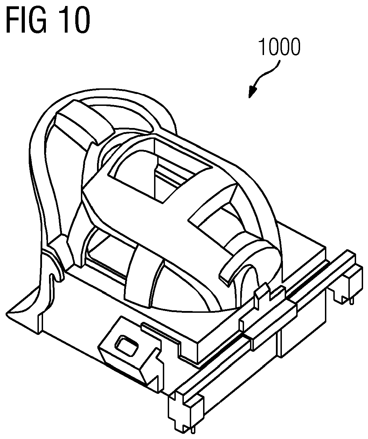

Weiterhin kann das Ermitteln der Position der Lokalspulen 35A, 35B in einem Unterschritt 42-2-2 des Teilschritts 42-2 der Fig. 4 ein Anpassen eines geometrischen Modells der Lokalspule 35A, 35B, beispielsweise eines in Fig. 10 gezeigten geometrischen Modells 1000 der Kopfspule, an die Abweichungen zwischen der ersten Tiefenkarte 801, 803 und der zweiten Tiefenkarte 802, 804 umfassen.

**Fig. 10** zeigt ein mögliches geometrisches Modell 1000 einer Lokalspule 35B zum Anpassen an die Abweichungen zwischen der ersten Tiefenkarte und der zweiten Tiefenkarte. In Fig. 10 ist dabei ein exemplarisches geometrisches Modell 1000 der zweiten Lokalspule 35B gemäß Fig. 8 und Fig. 9, also der Kopfspule, gezeigt.

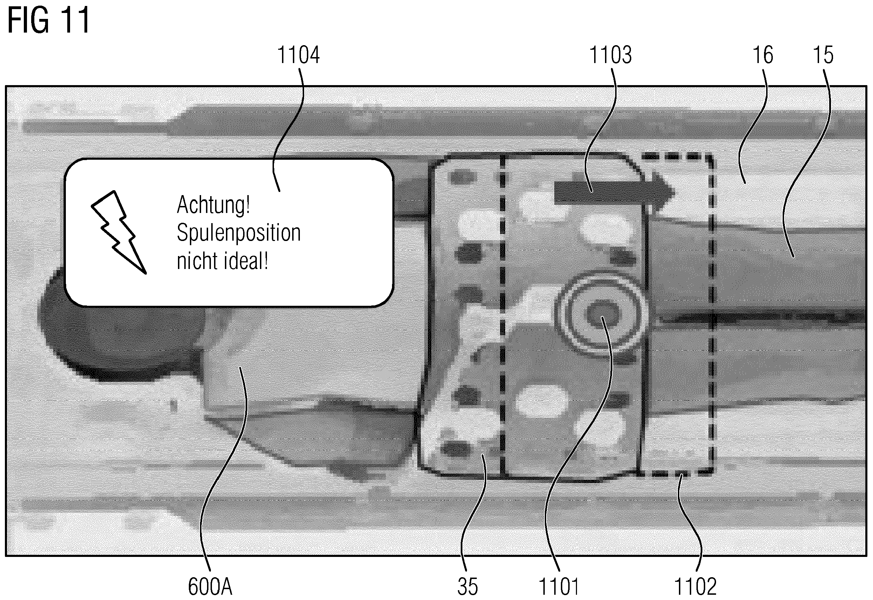

**Fig. 11** zeigt eine exemplarische Ausgabe eines Vorschlags 1103, 1104 zur Repositionierung einer Lokalspule 35. Dieser Vorschlag 1103, 1104 kann beispielsweise gemäß Fig. 5 im Teilschritt 43-3 des weiteren Verfahrensschritts 43 ausgegeben werden.

Im in Fig. 11 gezeigten Fall ist das Untersuchungsobjekt 15 auf der Patientenlagerungsvorrichtung 16 zusammen mit einer als flexible Oberflächenspule ausgebildeten Lokalspule 35 gelagert. An das Untersuchungsobjekt 15 wurde ein Körpermodell 600A angepasst, um die höchste Lokalisierungswahrscheinlichkeit 1101 des zu untersuchenden Organs, im in Fig. 11 gezeigten Fall der Prostata, zu bestimmen. Die höchste Lokalisierungswahrscheinlichkeit 1101 ist in Fig. 11 in Form von Konfidenzradien auf das Untersuchungsobjekt 15 projiziert.

In Fig. 11 ist die aktuelle Position 1101 der Lokalspule 35 bereits in der Tiefenkarte erkannt worden. Es ist deutlich, dass die Lokalspule 35 in ihrer aktuellen Position 1101 nicht zentriert über der höchsten Lokalisierungswahrscheinlichkeit 1101 der Prostata positioniert ist. Vielmehr ist bereits anhand der höchsten Lokalisierungswahrscheinlichkeit 1101 eine gewünschte Position 1102 für die Lokalspule 35 berechnet worden, wie im weiteren Teilschritt 42-4 des weiteren Verfahrensschritts 42 der Fig. 5 beschrieben.

Ein Vergleich der ermittelten aktuellen Position 1101 der Lokalspule 35 mit der gewünschten Position 1102 für die Lokalspule 1102 ist, wie im weiteren Teilschritt 42-5 des weiteren Verfahrensschritts 42 der Fig. 5, durchgeführt worden. Der Vergleich ergibt, dass die Lokalspule 35 in Richtung der Füße des Untersuchungsobjekts 15 repositioniert werden muss, wobei das Zentrum der Lokalspule 35 direkt über der höchsten Lokalisierungswahrscheinlichkeit 1101 der Prostata gelagert werden sollte.

Dementsprechend kann ein geeigneter Vorschlag 1103, 1104 zur Repositionierung der Lokalspule 35 an das Bedienpersonal ausgegeben werden. In Fig. 11 sind exemplarisch zwei Möglichkeiten für den Vorschlag 1103, 1104 eingezeichnet. Selbstverständlich können diese beiden Möglichkeiten auch getrennt voneinander eingesetzt werden und andere Möglichkeiten für den Vorschlag sind auch denkbar. Gemäß Fig. 11 sieht die erste Möglichkeit für den Vorschlag 1103 die Projektion bzw. Anzeige eines Verschiebungsvektors vor. Die zweite Möglichkeit für den Vorschlag 1104 sieht die Ausgabe eines Warnhinweises an das Bedienpersonal vor.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den durch die Ansprüche definierten Schutzumfang der Erfindung zu verlassen.

## Patentansprüche

1. Verfahren zum Bereitstellen einer Vorbereitungsinformation zur Vorbereitung einer Magnetresonanz-Bildgebung eines Untersuchungsobjekts mittels eines Magnetresonanzgeräts (11), umfassend folgende Verfahrensschritte:
- Lagerung des Untersuchungsobjekts auf einer Patientenlagerungsvorrichtung (16) des Magnetresonanzgeräts,
- Erfassen einer Tiefenkarte des auf der Patientenlagerungsvorrichtung gelagerten Untersuchungsobjekts mittels einer Tiefenkamera (36),
- Ermitteln einer Vorbereitungsinformation zur Vorbereitung der Magnetresonanz-Bildgebung unter Verwendung der erfassten Tiefenkarte und
- Bereitstellen der Vorbereitungsinformation,
**dadurch gekennzeichnet, dass** das Ermitteln der Vorbereitungsinformation ein Berechnen einer Lokalisierungswahrscheinlichkeit eines in der Magnetresonanz-Bildgebung zu untersuchenden Organs in einem Körper des Untersuchungsobjekts unter Verwendung der Tiefenkarte umfasst, wobei das Berechnen der Lokalisierungswahrscheinlichkeit des zu untersuchenden Organs eine Anpassung eines Körpermodells (600), welches eine Information über eine Lokalisierung des zu untersuchenden Organs im Körpermodell umfasst, an das Untersuchungsobjekt unter Verwendung der Tiefenkarte umfasst.

2. Verfahren nach Anspruch 1, wobei das Bereitstellen der Vorbereitungsinformation eine Anzeige von Anzeigedaten, welche basierend auf der Lokalisierungswahrscheinlichkeit des zu untersuchenden Organs erstellt werden, auf einer Anzeigeeinheit zusammen mit einer Darstellung einer Repräsentation eines menschlichen Körpers umfasst.

3. Verfahren nach einem der vorhergehenden Ansprüche, wobei das Bereitstellen der Lokalisierungswahrscheinlichkeit eine Projektion von Projektionsdaten, welche basierend auf der Lokalisierungswahrscheinlichkeit des zu untersuchenden Organs erstellt werden, auf eine Körperoberfläche des Untersuchungsobjekts mittels einer Projektionseinheit umfasst.

4. Verfahren nach Anspruch 3, wobei als Projektionsdaten zumindest ein Konfidenzradius auf die Körperoberfläche des Untersuchungsobjekts projiziert wird, wobei der zumindest eine Konfidenzradius die Lokalisierungswahrscheinlich des zu untersuchenden Organs wiederspiegelt.

5. Verfahren nach einem der vorhergehenden Ansprüche, wobei anhand der ermittelten Lokalisierungswahrscheinlichkeit die Patientenlagerungsvorrichtung des Magnetresonanzgeräts derart verfahren wird, dass für die Magnetresonanz-Bildgebung das zu untersuchende Organ automatisch in einem Isozentrum des Magnetresonanzgeräts positioniert wird.

6. Verfahren nach einem der vorhergehenden Ansprüche, wobei die erfasste Tiefenkarte eine Lokalspule (35), welche zum Empfangen von Magnetresonanz-Signalen während der Magnetresonanz-Bildgebung des Untersuchungsobjekts vorgesehen ist, abbildet, wobei das Ermitteln der Vorbereitungsinformation ein Ermitteln einer Position der Lokalspule anhand der Tiefenkarte umfasst.

7. Verfahren nach Anspruch 6, wobei das Erfassen der Tiefenkarte ein Erfassen einer ersten Tiefenkarte zeitlich vor einer Positionierung der Lokalspule für die Magnetresonanz-Bildgebung und ein Erfassen einer zweiten Tiefenkarte zeitlich nach der Positionierung der Lokalspule für die Magnetresonanz-Bildgebung umfasst, wobei das Ermitteln der Position der Lokalspule eine Bestimmung von Abweichungen zwischen der ersten Tiefenkarte und der zweiten Tiefenkarte umfasst.

8. Verfahren nach Anspruch 7, wobei das Ermitteln der Position der Lokalspule ein Anpassen eines geometrischen Modells der Lokalspule an die Abweichungen zwischen der ersten Tiefenkarte und der zweiten Tiefenkarte umfasst.

9. Verfahren nach einem der Ansprüche 6-8, wobei ein Ermitteln der Positionen von mehreren Lokalspulen, welche zum Erfassen der Magnetresonanz-Signale während der Magnetresonanz-Bildgebung des Untersuchungsobjekts vorgesehen sind, anhand der Tiefenkarte erfolgt, wobei für das Ermitteln der Positionen der mehreren Lokalspulen unterschiedliche Marker, welche und auf einer Oberfläche der mehreren Lokalspulen angebracht sind, in einem mittels der Tiefenkamera akquirierten Bild erkannt werden.

10. Verfahren nach Anspruch 6, wobei das Ermitteln der Position der Lokalspule unter Verwendung eines Körpermodells erfolgt, welches an das Untersuchungsobjekt angepasst wird, wobei zum Ermitteln der Position der Lokalspule Abweichungen zwischen der Tiefenkarte und dem an das Untersuchungsobjekt angepassten Körpermodell bestimmt werden.

11. Verfahren nach einem der Ansprüche 6-8 und 10, wobei das Ermitteln der Vorbereitungsinformation einen Vergleich der ermittelten Position der Lokalspule mit einer gewünschten Position für die Lokalspule zum Empfangen von Magnetresonanz-Signalen aus dem zu untersuchenden Organ umfasst, wobei die gewünschte Position für die Lokalspule anhand der berechneten Lokalisierungswahrscheinlichkeit des zu untersuchenden Organs ermittelt wird.

12. Verfahren nach Anspruch 11, wobei das Bereitstellen der Vorbereitungsinformation eine Ausgabe eines Vorschlags zur Repositionierung der Lokalspule basierend auf einem Ergebnis des Vergleichs umfasst.

13. System, umfassend ein Magnetresonanzgerät (11) mit einer Patientenlagerungsvorrichtung (16), eine Tiefenkamera (36) und eine Recheneinheit (24), wobei das System zum Ausführen eines Verfahrens nach einem der vorhergehenden Ansprüche ausgebildet ist.

14. Computerprogrammprodukt, welches direkt in einen Speicher einer programmierbaren Recheneinheit ladbar ist, mit Programmcode-Mitteln, um mittels des Systems aus Anspruch 13 die Verfahrensschritte, welche sich an die Lagerung des Untersuchungungsobjekts in dem Verfahren nach einem der Ansprüche 1-12 anschliessen, auszuführen, wenn das Computerprogrammprodukt in der Recheneinheit ausgeführt wird.

## Claims

1. Method for provision of preparatory information for preparation of magnetic resonance imaging of an examination object by means of a magnetic resonance device (11), comprising the following method steps:
- Supporting of the examination object on a patient support facility (16) of the magnetic resonance device,
- Acquisition of a depth map of the examination object supported on the patient support facility by means of a time-of-flight camera (36),
- Establishment of a preparatory information for the preparation of the magnetic resonance imaging using the acquired depth map and
- Provision of the preparatory information,
**characterised in that** the establishment of the preparatory information comprises a computation of a probability of localisation of an organ in a body of the examination object to be examined in the magnetic resonance imaging while using the depth map, wherein the computation of the probability of localisation of the organ to be examined comprises a matching of a body model (600), which comprises information about a localisation of the organ to be examined in the body model, to the examination object while using the depth map.

2. Method according to claim 1, wherein the provision of the preparatory information comprises a display of display data, which is created based on the probability of localisation of the organ to be examined, on a display unit together with a display of a representation of a human body.

3. Method according to one of the preceding claims, wherein the provision of the probability of localisation comprises a projection of projection data, which is created based on the probability of localisation of the organ to be examined, onto a body surface of the examination object by means of a projection unit.

4. Method according to claim 3, wherein at least one confidence radius is projected on the body surface of the examination object as projection data, wherein the at least one confidence radius reflects the localisation probability of the organ to be examined.

5. Method according to one of the preceding claims, wherein, on the basis of the established probability of localisation, the patient support facility of the magnetic resonance device is moved such that, for the magnetic resonance imaging, the organ to be examined is positioned automatically in an isocentre of the magnetic resonance device.

6. Method according to one of the preceding claims, wherein the acquired depth map maps a local coil (35), which is provided for receiving magnetic resonance signals during the magnetic resonance imaging of the examination object, wherein the establishment of the preparatory information comprises establishing a position of the local coil on the basis of the depth map.

7. Method according to claim 6, wherein the acquisition of the depth map comprises an acquisition of a first depth map in the time before a positioning of the local coil for the magnetic resonance imaging and an acquisition of a second depth map in the time after the positioning of the local coil for the magnetic resonance imaging, wherein the establishment of the position of the local coil comprises a determination of differences between the first depth map and the second depth map.

8. Method according to claim 7, wherein the establishment of the position of the local coil comprises a matching of a geometrical model of the local coil to the differences between the first depth map and the second depth map.

9. Method according to one of claims 6 - 8, wherein an establishment of the positions of a number of local coils, which are provided for acquisition of the magnetic resonance signals during the magnetic resonance imaging of the examination object, is done on the basis of the depth map, wherein for the establishment of the positions of the number of local coils, different markers, which are attached to a surface of the number local coils, are recognised in an image acquired by means of the time-of-flight camera.

10. Method according to claim 6, wherein the position of the local coil is established using a body model, which is matched to the examination object, wherein for establishing the position of the local coil, differences between the depth map and the body model matched to the examination object are determined.

11. Method according to one of claims 6 - 8 and 10, wherein the establishment of the preparatory information comprises a comparison between the established position of the local coil and a desired position for the local coil for receiving magnetic resonance signals from the organ to be examined, wherein the desired position for the local coil is established on the basis of the computed probability of localisation of the organ to be examined.

12. Method according to claim 11, wherein the provision of the preparatory information comprises an output of a suggestion for repositioning of the local coil based on a result of the comparison.

13. System, comprising a magnetic resonance device (11) with a patient support facility (16), a time-of-flight camera (36) and a computing unit (24), wherein the system is embodied for carrying out a method according to one of the preceding claims.

14. Computer program product, which is able to be loaded directly into a memory of a programmable computing unit, with program code means for carrying out the method steps, by means of the system from claim 13, which are affiliated with the support of the examination object in the method according to one of claims 1 - 12, when the computer program product is executed in the computing unit.

## Revendications

1. Procédé pour donner une information de préparation pour préparer une imagerie par résonance magnétique d'un objet à examiner au moyen d'un appareil (11) à résonance magnétique, comprenant les stades de procédé suivants :
- on met l'objet à examiner sur une installation (16) de mise en position d'un patient de l'appareil à résonance magnétique,
- on relève, au moyen d'un appareil (36) photographique de profondeur, une carte de profondeur de l'objet à examiner mis sur le système de mise en position d'un patient,
- on détermine une information de préparation pour la préparation de l'imagerie par résonance magnétique en utilisant la carte de profondeur relevée et
- on donne l'information de préparation,
**caractérisé en ce que** la détermination de l'information de préparation comprend un calcul d'une probabilité de localisation d'un organe, à examiner dans l'imagerie par résonance magnétique, dans un corps de l'objet à examiner, en utilisant la carte de profondeur, le calcul de la probabilité de localisation de l'organe à examiner comprenant une adaptation d'un modèle (600) du corps, qui comprend une information sur une localisation de l'organe à examiner dans le modèle du corps, à l'objet à examiner en utilisant la carte de profondeur.

2. Procédé suivant la revendication 1, dans lequel, donner l'information de préparation comprend un affichage de données d'affichage sur une unité d'affichage, qui sont établies en se fondant sur la probabilité de localisation de l'organe à examiner, ensemble avec visualisation d'une représentation d'un corps humain.

3. Procédé suivant l'une des revendications précédentes, dans lequel donner la probabilité de localisation comprend une projection, sur une surface du corps de l'objet à examiner, au moyen d'une unité de projection, de données de projection, qui sont établies en se fondant sur la probabilité de localisation de l'organe à examiner.

4. Procédé suivant la revendication 3, dans lequel on projette, comme données de projection, au moins un rayon de confiance sur la surface du corps de l'objet à examiner, le au moins un rayon de confiance reflétant la probabilité de localisation de l'organe à examiner.

5. Procédé suivant l'une des revendications précédentes, dans lequel, à l'aide de la probabilité de localisation déterminée, on déplace le système de mise en position du patient de l'appareil à résonance magnétique, de manière à mettre, pour l'imagerie par résonance magnétique, l'organe à examiner en position automatiquement à un iso-centre de l'appareil à résonance magnétique.

6. Procédé suivant l'une des revendications précédentes, dans lequel la carte de profondeur relevée représente une bobine (35) locale, qui est prévue pour la réception de signaux de résonance magnétique pendant l'imagerie par résonance magnétique de l'objet à examiner, la détermination de l'information de préparation comprenant une détermination d'une position de la bobine locale à l'aide de la carte de profondeur.

7. Procédé suivant la revendication 6, dans lequel le relevé de la carte de profondeur comprend un relevé d'une première carte de profondeur avant, dans le temps, une mise en position de la bobine locale pour l'imagerie par résonance magnétique, et un relevé d'une deuxième carte de profondeur après, dans le temps, la mise en position de la bobine locale pour l'imagerie par résonance magnétique, la détermination de la position de la bobine locale comprenant une détermination d'écarts entre la première carte de profondeur et la deuxième carte de profondeur.

8. Procédé suivant la revendication 7, dans lequel la détermination de la position de la bobine locale comprend une adaptation d'un modèle géométrique de la bobine locale aux écarts entre la première carte de profondeur et la deuxième carte de profondeur.

9. Procédé suivant l'une des revendications 6 à 8, dans lequel il est produit, à l'aide de la carte de profondeur, une détermination des positions de plusieurs bobines locales, qui sont prévues pour le relevé des signaux de résonance magnétique pendant l'imagerie par résonance magnétique de l'objet à examiner, dans lequel, pour la détermination des positions des plusieurs bobines locales, des repérages différents, qui sont mis sur une surface des plusieurs bobines locales, sont détectés dans une image acquise au moins de l'appareil photographique de profondeur.

10. Procédé suivant la revendication 6, dans lequel on effectue la détermination de la position de la bobine locale en utilisant un modèle de corps, qui est adapté à l'objet à examiner, dans lequel, pour la détermination de la position de la bobine locale, on détermine des écarts entre la carte de profondeur et le modèle de corps adapté à l'objet à examiner.

11. Procédé suivant l'une des revendications 6 à 8 et 10, dans lequel la détermination de l'information de préparation comprend une comparaison de la position déterminée de la bobine locale à une position souhaitée de la bobine locale pour la réception de signaux de résonance magnétique à partir de l'organe à examiner, dans lequel on détermine la position souhaitée de la bobine locale à l'aide de la probabilité de localisation calculée de l'organe à examiner.

12. Procédé suivant la revendication 11, dans lequel donner l'information de préparation comprend une émission d'une proposition de repositionnement de la bobine locale sur la base d'un résultat de la comparaison.

13. Système, comprenant un appareil (11) de résonance magnétique, ayant une installation (16) de mise en position d'un patient, un appareil (36) photographique de profondeur et une unité (24) informatique,
le système étant constitué pour l'exécution d'un procédé suivant l'une des revendications précédentes.

14. Produit de programme d'ordinateur, qui peut être chargé directement dans une mémoire de l'unité de calcul programmable par des moyens de code de programme, pour effectuer, au moyen du système de la revendication 13, les stades de procédé, qui font suite à la mise en position de l'objet à examiner dans le procédé suivant l'une des revendications 1 à 12, lorsque le produit de programme d'ordinateur est réalisé dans l'unité informatique.
